# EUROPEAN PATENT APPLICATION

(11) **EP 4 722 345 A1**
(43) Date of publication of application: **08.04.2026**
(21) Application number: 24815499.9
(22) Date of filing: 29.05.2024
(51) Int. Cl.: C12N 5/077, C12N 5/10

(54) **METHOD FOR MATURING CARDIOMYOCYTES, AND METHOD FOR PRODUCING MATURED CARDIOMYOCYTES**

(30) Priority: 29.05.2023 JP 2023087929
(71) Applicant: RIKEN, Wako-shi, Saitama 351-0198 (JP)
(72) Inventor: MASUMOTO, Hidetoshi, Wako-shi, Saitama 351-0198 (JP); MAIHEMUTI, Wusiman, Wako-shi, Saitama 351-0198 (JP); MURATA, Kozue, Wako-shi, Saitama 351-0198 (JP)
(74) Representative: Maiwald GmbH
(86) International application number: PCT/JP2024/019642
(87) International publication number: WO 2024/248017

(57) **Abstract**

A method of producing matured cardiomyocytes, the method comprising: a preparation step to prepare a sample containing immature cardiomyocytes; and a pressure-applying step to apply hydrostatic pressure continuously or intermittently to the sample.

## Description

### TECHNICAL FIELD

The present invention relates to a method of maturing cardiomyocytes and a method of producing matured cardiomyocytes.

### BACKGROUND ART

Cardiomyocytes of an adult hardly proliferate and, therefore, loss of cardiomyocytes due to ischemic heart diseases and/or other causes is a damage that is irreversible. No treatment with conventional drugs used in clinical settings can effectively replace a myocardial scar by a functional contractile tissue. Thus, there has been a demand for novel therapies for regenerating normal cardiomyocytes, and there has been a suggestion for replacement therapy or transplantation therapy that involves administration of new cardiomyocytes produced by induced differentiation from pluripotent stem cells (e.g., human iPS cells).

For example, Japanese National Patent Publication No. 2016-536975 (PTL 1) discloses a method for bioengineering cardiac muscle (bioengineered heart muscle, BHM) from pluripotent stem cells, in which the method generally comprises a step to induce mesoderm differentiation, heart differentiation, and heart maturation by directed histogenesis.

In addition, International Patent Laying-Open No. WO 2017/073794 (PTL 2) discloses a method for producing myocardial tissue comprising (a) a step to produce cardiomyocytes from pluripotent stem cells, (b) a step to produce endothelial cells from pluripotent stem cells, (c) a step to produce mural cells from pluripotent stem cells, (d) a step to mix the mural cells produced in the step (c) in a proportion of less than 30% with the cardiomyocytes produced in the step (a) and the endothelial cells produced in the step (b), and (e) a step to incubate the cell mixture produced in the step (d) in the presence of an extracellular matrix to form a three-dimensional structure.

### CITATION LIST

### PATENT LITERATURE

PTL 1: Japanese National Patent Publication No. 2016-536975
PTL 2: International Patent Laying-Open No. WO 2017/073794

### NON PATENT LITERATURE

NPL 1: Methods Mol. Biol. 2021; 2320:81-88.

### SUMMARY OF INVENTION

### TECHNICAL PROBLEM

Cardiomyocytes (CMs) differentiated from human pluripotent stem cells (hPSCs) (hereinafter such CMs are also referred to as "hPSC-CMs") are similar to early-stage human CMs (immature CMs in human embryos or fetuses) in many ways. However, hPSC-CMs are different from matured human CMs in function and structure. In clinical applications of hPSC-CMs (e.g., when using them in replacement therapy, transplantation, and the like), the cells need to be the same as or similar to matured human CMs in function and structure.

Although various methods have been suggested for producing matured human CMs, those methods have limited effect to recover myocardial function.

The present invention has been devised in light of the above-described circumstances, and an object of the present invention is to provide a method of efficiently maturing cardiomyocytes in vitro as well as a method of producing matured cardiomyocytes in vitro.

### SOLUTION TO PROBLEM

The inventors of the present invention have conducted intensive research, and as a result, have found that application of a certain amount of hydrostatic pressure to a sample containing immature cardiomyocytes can efficiently mature the cardiomyocytes. Thus, the present invention has now been completed. In the following, the present invention will be described in more detail.
[1] A method of maturing cardiomyocytes, the method comprising:
   a preparation step to prepare a sample containing immature cardiomyocytes; and
   a pressure-applying step to apply hydrostatic pressure continuously or intermittently to the sample.
[2] The method of maturing cardiomyocytes according to [1], wherein the hydrostatic pressure applied in the pressure-applying step is from 10 kPa to 100 kPa.
[3] The method of maturing cardiomyocytes according to [1] or [2], wherein in the pressure-applying step, the hydrostatic pressure is applied intermittently at a frequency from 1 hour to 12 hours per day.
[4] The method of maturing cardiomyocytes according to any one of [1] to [3], wherein the pressure-applying step is implemented for a period from 3 days to 8 days.
[5] The method of maturing cardiomyocytes according to any one of [1] to [4], wherein the sample further contains vascular mural cells.
[6] The method of maturing cardiomyocytes according to any one of [1] to [5], wherein the sample further contains vascular endothelial cells.
[7] The method of maturing cardiomyocytes according to any one of [1] to [6], wherein the sample is an engineered cardiac tissue derived from iPS cells.
[8] An engineered cardiac tissue containing cardiomyocytes, wherein
   the cardiomyocytes include TNNI3-positive cells.
[9] A method of producing matured cardiomyocytes, the method comprising:
   a preparation step to prepare a sample containing immature cardiomyocytes; and
   a pressure-applying step to apply hydrostatic pressure continuously or intermittently to the sample.
[10] The method of producing matured cardiomyocytes according to [9], wherein the hydrostatic pressure applied in the pressure-applying step is from 10 kPa to 100 kPa.
[11] The method of producing matured cardiomyocytes according to [9] or [10], wherein in the pressure-applying step, the hydrostatic pressure is applied intermittently at a frequency from 1 hour to 12 hours per day.
[12] The method of producing matured cardiomyocytes according to any one of [9] to [11], wherein the pressure-applying step is implemented for a period from 3 days to 8 days.
[13] The method of producing matured cardiomyocytes according to any one of [9] to [12], wherein the sample further contains vascular mural cells.
[14] The method of producing matured cardiomyocytes according to any one of [9] to [13], wherein the sample further contains vascular endothelial cells.
[15] The method of producing matured cardiomyocytes according to any one of [9] to [14], wherein the sample is an engineered cardiac tissue derived from iPS cells.

### ADVANTAGEOUS EFFECTS OF INVENTION

The present invention makes it possible to provide a method of efficiently maturing cardiomyocytes in vitro as well as a method of producing matured cardiomyocytes in vitro.

### BRIEF DESCRIPTION OF DRAWINGS

[Fig. 1] Fig. 1 shows (A) a graph of the cell composition of an engineered cardiac tissue (ECT), and (B) photographs of the ECT.
[Fig. 2] Fig. 2 shows a graph of the "cycle" for applying hydrostatic pressure.
[Fig. 3] Fig. 3 shows a schematic view of the schedule for applying hydrostatic pressure.
[Fig. 4] Fig. 4 shows photographs of tissue staining of ECT.
[Fig. 5] Fig. 5 shows photographs of tissue staining of ECT.
[Fig. 6] Fig. 6 shows graphs of the amount of expression of genes in ECT.
[Fig. 7A] Fig. 7A shows photographs of tissue staining of ECT.
[Fig. 7BC] Fig. 7BC shows graphs of the number of cells in ECT, cTnT-positive ratio, and thickness.
[Fig. 7DE] Fig. 7DE shows graphs of FFR of ECT.
[Fig. 7FG] Fig. 7FG shows graphs of the amount of expression of genes in ECT.
[Fig. 7H] Fig. 7H shows photographs of results of fluorescence analysis of ECT.
[Fig. 8AB] Fig. 8AB shows graphs of FFR of ECT.

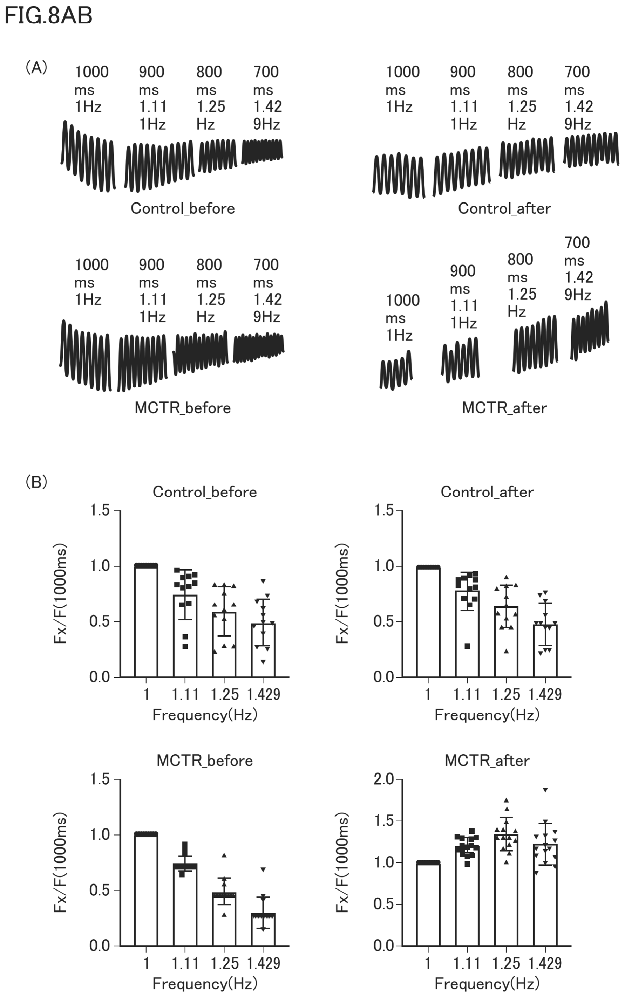

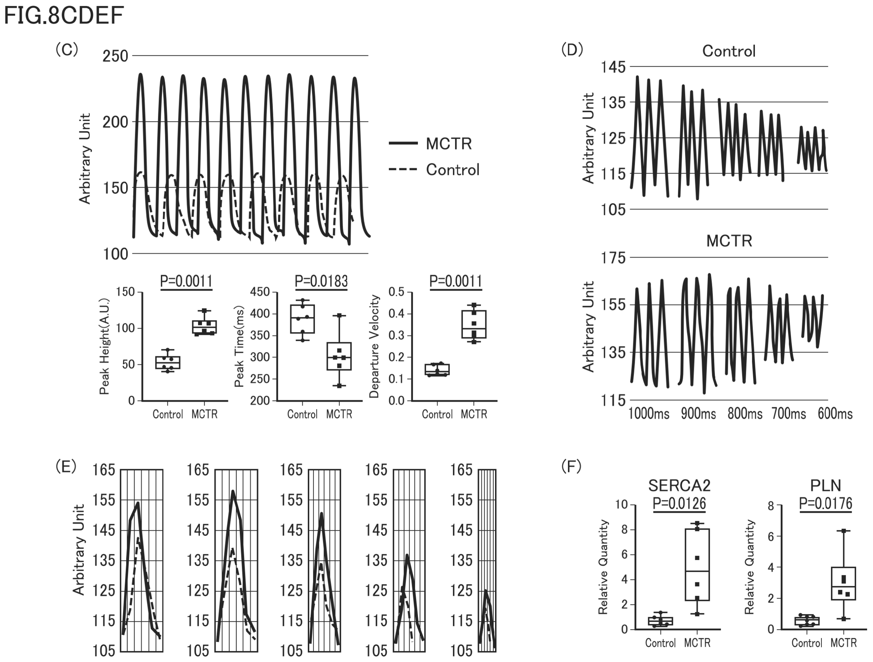

[Fig. 8CDEF] Fig. 8CDEF shows graphs of results of evaluation of calcium transient in ECT.

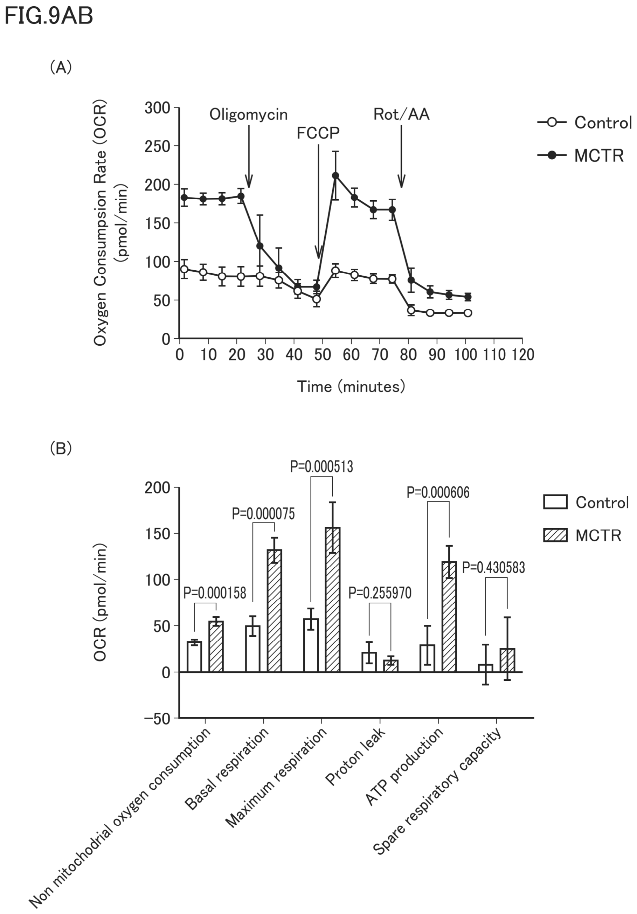

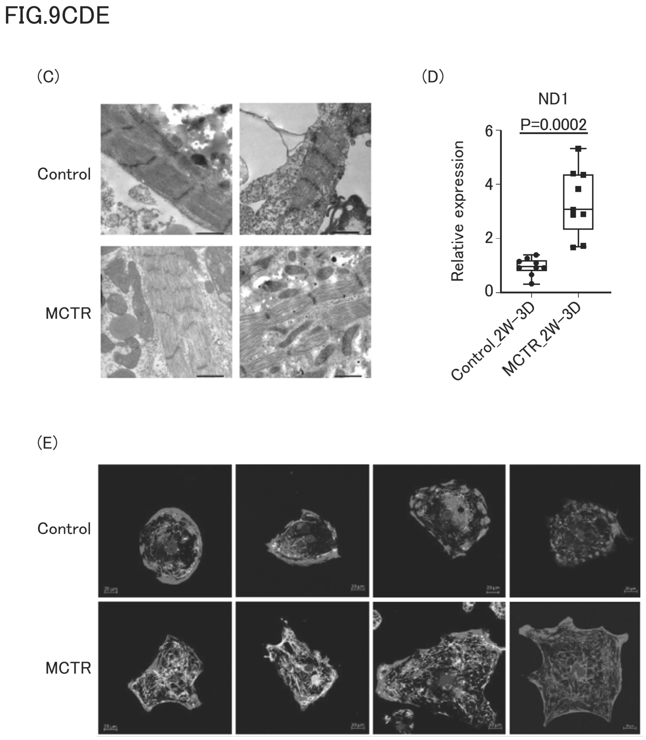

[Fig. 9AB] Fig. 9AB shows graphs of results of mitochondrial function (the maximum oxygen consumption rate) in ECT.

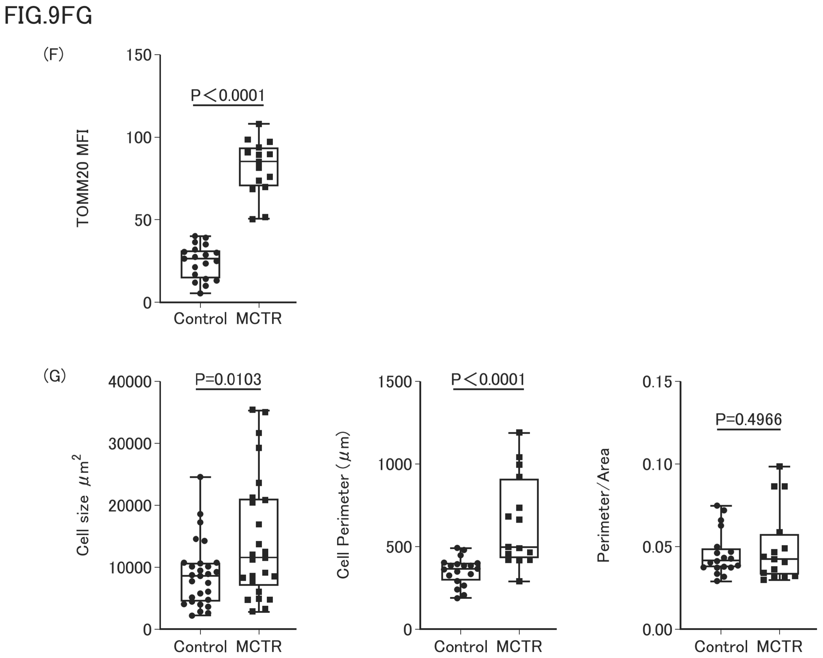

[Fig. 9CDE] Fig. 9CDE shows (C) photographs of mitochondria in ECT, (D) a graph of the amount of expression of genes in ECT, and (E) photographs of the amount of mitochondrial DNA.
[Fig. 9FG] Fig. 9FG shows (F) a graph of the amount of expression of TOMM20 in ECT, as well as (G) graphs of the cell size, the cell perimeter, and the ratio of cell perimeter to cell area.

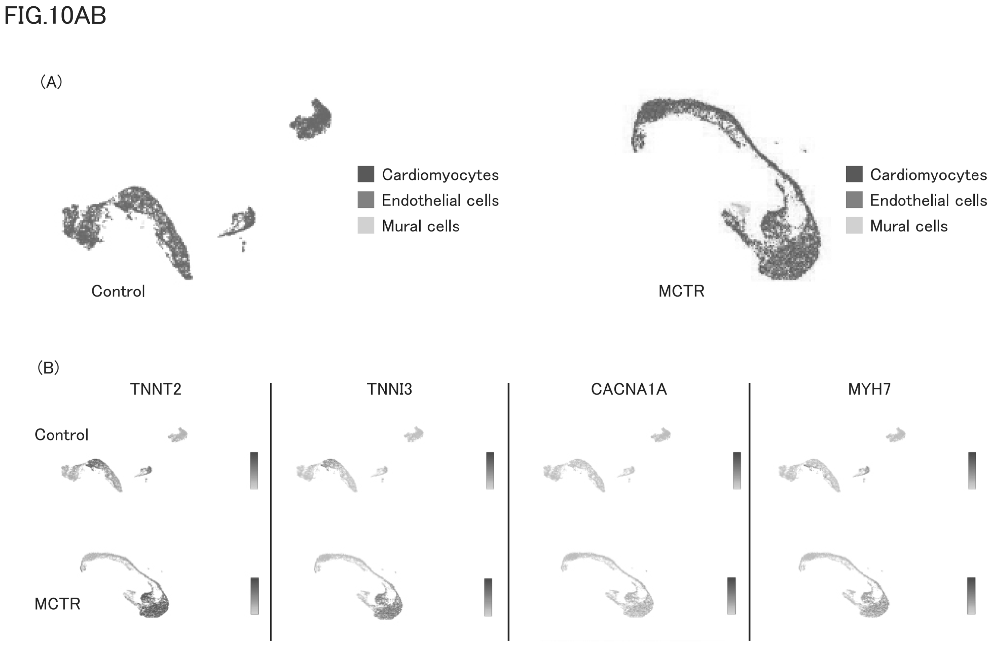

[Fig. 10AB] Fig. 10AB shows images of results of single cell RNA-seq of ECT.

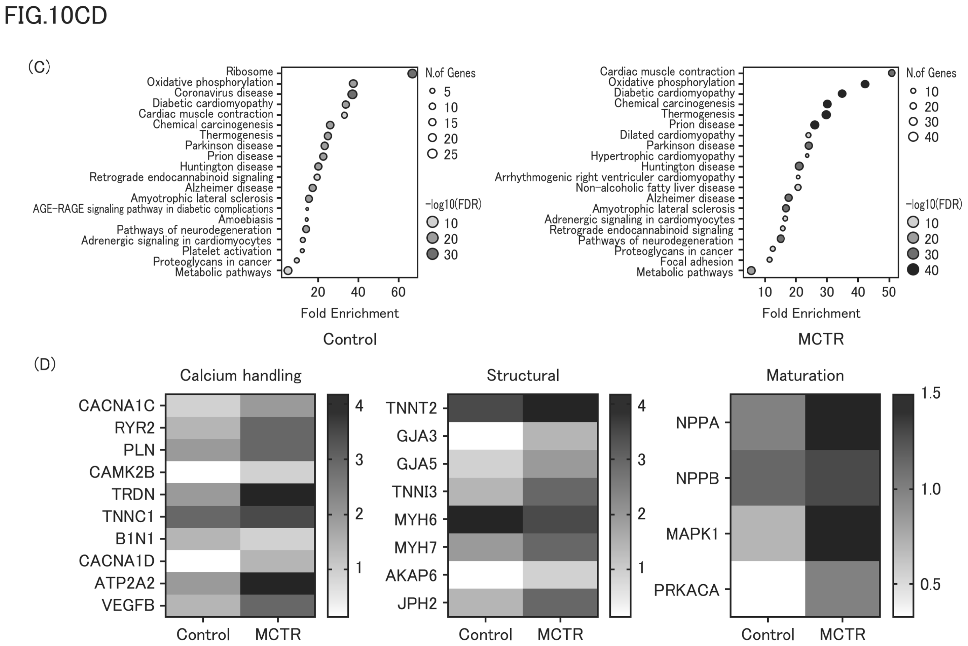

[Fig. 10CD] Fig. 10CD shows graphs of results of single cell RNA-seq of ECT.

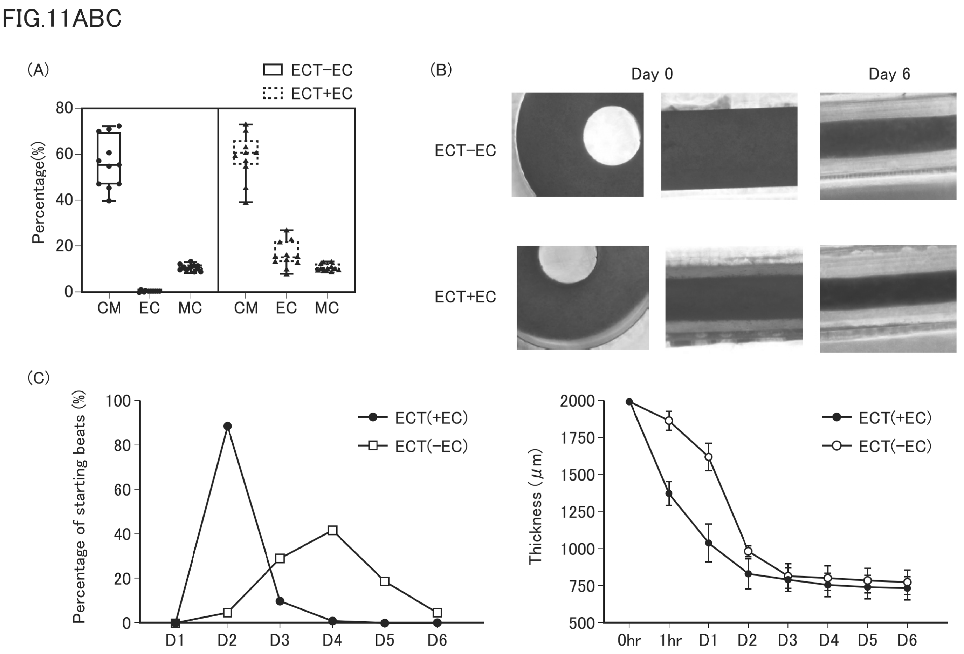

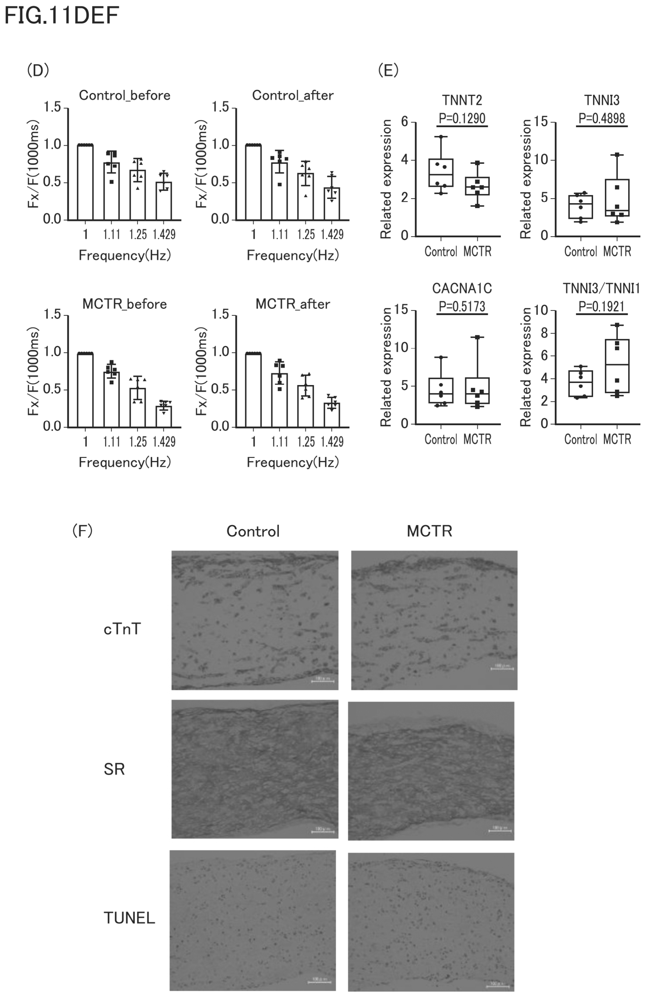

[Fig. 11ABC] Fig. 11ABC shows (A) a graph of the cell composition of an ECT, (B) photographs of the ECT, as well as (C) graphs of the percentage of beats of the ECT and the thickness.
[Fig. 11DEF] Fig. 11DEF shows (D) graphs of FFR of ECT, (E) graphs of the amount of expression of genes, and (F) photographs of tissue staining of ECT.

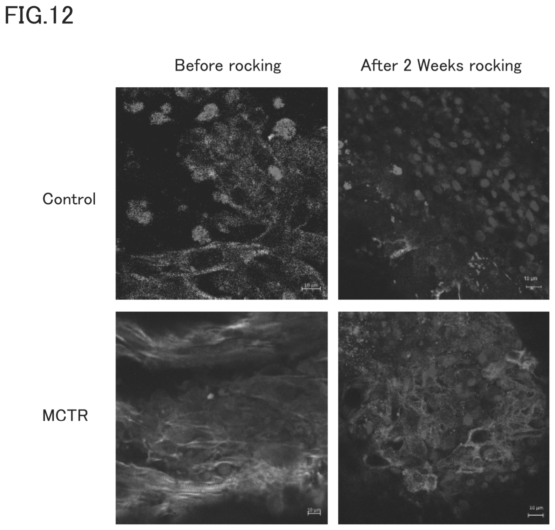

[Fig. 12] Fig. 12 shows images of results of confocal imaging of ECT.

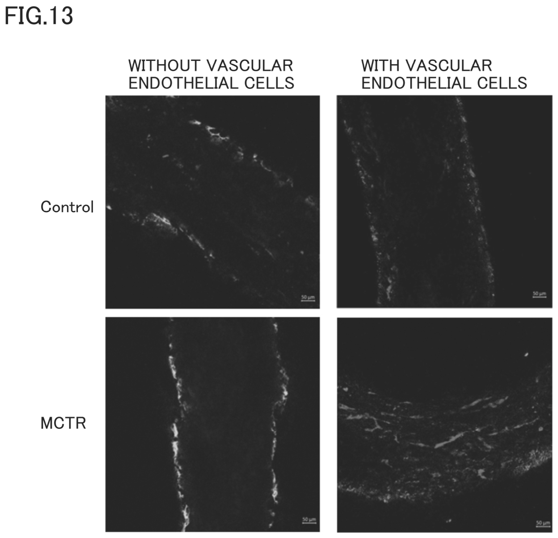

[Fig. 13] Fig. 13 shows images of results of confocal imaging of ECT.

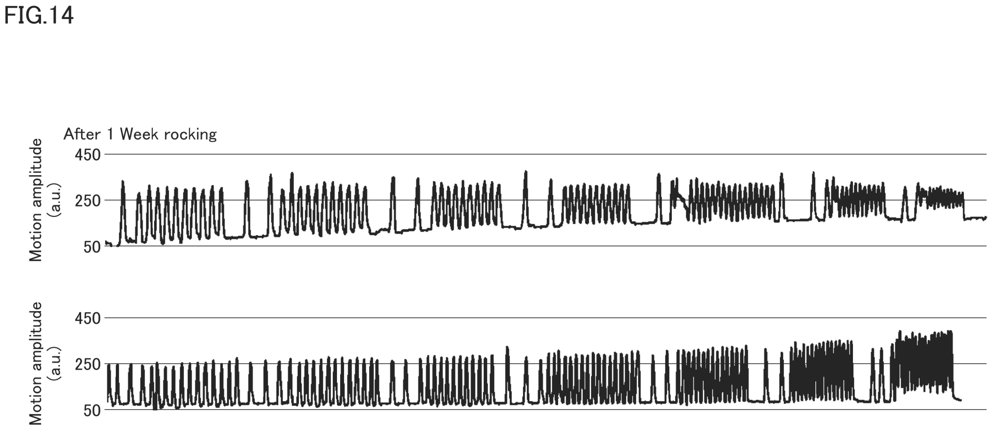

[Fig. 14] Fig. 14 shows graphs of FFR of ECT.

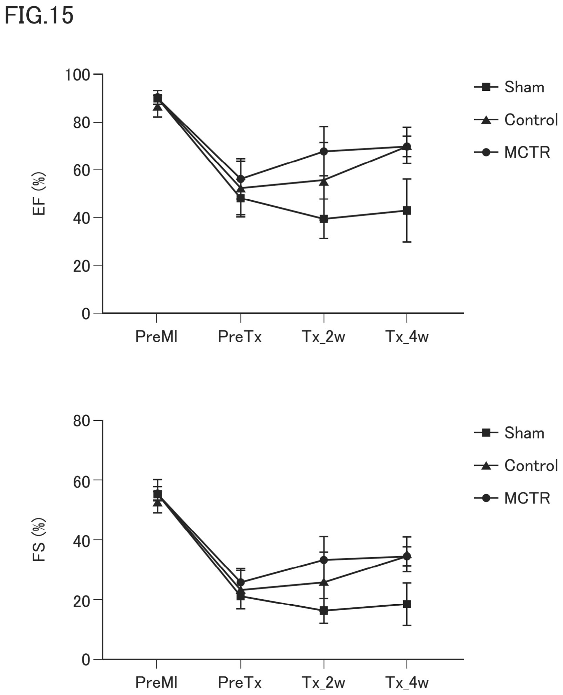

[Fig. 15] Fig. 15 shows graphs of results of echocardiography. The vertical axis represents ejection fraction (EF) or left ventricular fractional shortening (FS), and the horizontal axis represents sample names.

### DESCRIPTION OF EMBODIMENTS

In the following, a description will be given of an embodiment of the present invention (hereinafter referred to as "the present embodiment"). It should be noted that the present embodiment is not limited to the below description. In the present specification, the expression "(from) A to Z" means the upper limit and the lower limit of a range (that is, it means not less than A and not more than Z), and if a description of unit is attached only to Z but not to A, A has the same unit as that of Z.

### <<Method of Maturing Cardiomyocytes>>

A method of maturing cardiomyocytes according to the present embodiment comprises:
a preparation step to prepare a sample containing immature cardiomyocytes; and
a pressure-applying step to apply hydrostatic pressure continuously or intermittently to the sample.

### <Preparation Step>

In this step, a sample containing immature cardiomyocytes is prepared. In the present embodiment, "cardiomyocytes" means myocytes that constitute cardiac muscle and express at least myocardial troponin (cTnT) or αMHC. cTnT of humans may be NCBI Accession Number NM_000364, and that of mice may be NM_001130174. αMHC of humans may be NCBI Accession Number NM_002471, and that of mice may be NM_001164171. "Immature cardiomyocytes" means cardiomyocytes immediately after differentiation from pluripotent stem cells and positive for TNNI1. Other examples of markers for immature cardiomyocytes include MYH6 and the like.

In the present embodiment, the sample is not particularly limited as long as it contains immature cardiomyocytes, and, for example, it may be in a state of cell suspension, or may be in a state of cell aggregate, or may be in a state of a three-dimensional structure. Examples of the three-dimensional structure include an engineered cardiac tissue described below. The shape of the three-dimensional structure is not particularly limited, and examples thereof include dumbbell shape, belt shape, string shape, sheet shape, donut shape, round, columnar, cylindrical, and the like.

### (Cardiomyocytes)

The immature cardiomyocytes can be produced by induced differentiation from pluripotent stem cells, for example. Examples of the pluripotent stem cells include the cells described below.

### (A) Embryonic Stem Cells

Embryonic stem cells (ES cells) are stem cells that are established from the inner cell mass of a mammalian (e.g., human or mouse) early embryo (e.g., blastocyst), and that have pluripotency and proliferative potential based on self-replication.

ES cells are embryo-derived stem cells derived from the inner cell mass of a blastocyst which is a stage of an embryo (a fertilized egg) after both the 8-cell stage and the morula stage, and ES cells have the ability to differentiate into any cells that constitute an adult body, the so-called pluripotency, as well as proliferative potential based on self-replication. ES cells were discovered in mice in 1981 (M. J. Evans and M. H. Kaufman (1981), Nature 292:154-156), and then ES cell lines were established for primates such as humans and monkeys (J. A. Thomson et al. (1998), Science 282:1145-1147; J. A. Thomson et al. (1995), Proc. Natl. Acad. Sci. USA, 92:7844-7848; J. A. Thomsonet al. (1996), Biol. Reprod., 55:254-259; J. A. Thomson and V.S. Marshall (1998), Curr. Top. Dev. Biol., 38:133-165).

ES cells can be established by isolating the inner cell mass from a blastocyst developed from a fertilized egg of an animal and incubating the inner cell mass on feeder fibroblasts. The cells can be maintained by passaging with the use of a culture medium supplemented with a substance such as leukemia inhibitory factor (LIF) and/or basic fibroblast growth factor (bFGF). The method for establishing and maintaining human and monkey ES cells is described in, for example, USP5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; and Klimanskaya I, et al. (2006), Nature. 444:481-485.

As the culture medium for preparing ES cells, DMEM/F-12 culture medium supplemented with 0.1 mM 2-mercaptoethanol, 0.1 mM non-essential amino acid, 2 mM L-glutamic acid, 20% KSR, and 4 ng/ml bFGF can be used, for example, and human ES cells can be maintained in a wet atmosphere at 37°C and 5% CO₂ (H. Suemori et al. (2006), Biochem. Biophys. Res. Commun., 345:926-932). ES cells need to be passaged every 3 to 4 days, and passaging can be carried out by using 0.25% trypsin and 0.1 mg/ml collagenase IV in PBS containing 1 mM CaCl₂ and 20% KSR, for example.

Typically, ES cells can be selected by Real-Time PCR, by using expression of a genetic marker such as alkaline phosphatase, Oct-3/4, and/or Nanog as an index. For selecting human ES cells, in particular, expression of a genetic marker such as OCT-3/4, NANOG, and/or ECAD can be used as an index (E. Kroon et al. (2008), Nat. Biotechnol., 26:443-452).

As a human ES cell line, WA01 (H1) and WA09 (H9), for example, are available from WiCell Research Institute. KhES-1, KhES-2, and KhES-3 are available from the Institute for Frontier Life and Medical Sciences (inFront), Kyoto University (Kyoto, Japan).

### (B) Spermatogonial Stem Cells

Spermatogonial stem cells are pluripotent stem cells that are derived from the testis and from which spermatogenesis occurs. Like ES cells, spermatogonial stem cells can be induced to differentiate into cells of various lineages, and one of the characteristics is that when transplanted into a mouse blastocyst, a chimeric mouse can be produced (M. Kanatsu-Shinohara et al. (2003) Biol. Reprod., 69:612-616; K. Shinohara et al. (2004), Cell, 119:1001-1012). Spermatogonial stem cells can self-replicate in a culture medium containing glial cell line-derived neurotrophic factor (GDNF), and can be obtained by repeated passaging under culture conditions similar to those for ES cells (Masanori Takebayashi et al. (2008), Jikken Igaku (Experimental Medicine), vol. 26, No. 5 (Extra Edition), p. 41-46, Yodosha (Tokyo, Japan)).

### (C) Embryonic Germ Cells

Embryonic germ cells are cells established from primordial germ cells during the embryonic stage and have pluripotency similar to that of ES cells; embryonic germ cells can be established by incubating primordial germ cells in the presence of substances such as LIF, bFGF, and stem cell factors (Y. Matsui et al. (1992), Cell, 70:841-847; J. L. Resnick et al. (1992), Nature, 359:550-551).

### (D) Induced Pluripotent Stem Cells

Induced pluripotent stem (iPS) cells are somatic cell-derived engineered stem cells that can be prepared by introducing particular reprogramming factors in the form of DNA, RNA, or protein into somatic cells, and that have properties nearly equivalent to those of ES cells, such as, for example, pluripotency as well as proliferative potential based on self-replication (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al, Nat. Biotechnol. 26:101-106 (2008); International Patent Laying-Open No. WO 2007/069666). Each reprogramming factor may be in the form of a gene specifically expressed in ES cells, or a gene product or non-coding RNA thereof; or a gene playing an important role in maintaining the undifferentiated state of ES cells, or a gene product or non-coding RNA thereof; or a small molecule compound. Genes included in the reprogramming factors include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, Glis1, and the like, for example, and each of these reprogramming factors may be used alone, or may be used in combination. Examples of the combination of reprogramming factors include those described in WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO 2010/056831, WO2010/068955, WO2010/098419, WO2010/102267, WO 2010/111409, WO 2010/111422, WO2010/115050, WO2010/124290, WO2010/147395, WO2010/147612, Huangfu D, et al. (2008), Nat. Biotechnol., 26: 795-797, Shi Y, et al. (2008), Cell Stem Cell, 2: 525-528, Eminli S, et al. (2008), Stem Cells. 26:2467-2474, Huangfu D, et al. (2008), Nat Biotechnol. 26:1269-1275, Shi Y, et al. (2008), Cell StemCell, 3, 568-574, Zhao Y, et al. (2008), Cell Stem Cell, 3:475-479, Marson A, (2008), Cell Stem Cell, 3, 132-135, Feng B, et al. (2009), Nat Cell Biol. 11:197-203, R. L. Judson et al., (2009), Nat. Biotech., 27:459-461, Lyssiotis CA, et al. (2009), Proc Natl Acad Sci U S A. 106:8912-8917, Kim JB, et al. (2009), Nature. 461:649-643, Ichida JK, et al. (2009), Cell Stem Cell. 5:491-503, Heng JC, et al. (2010), Cell Stem Cell. 6:167-74, Han J, et al. (2010), Nature. 463:1096-100, Mali P, et al. (2010), Stem Cells. 28:713-720, and Maekawa M, et al. (2011), Nature. 474:225-9.

The reprogramming factors also include factors used for enhancing establishment efficiency, such as histone deacetylase (HDAC) inhibitors [e.g., small molecule inhibitors such as valproic acid (VPA), trichostatin A, sodium butyrate, MC1293, and M344, and nucleic acid-based expression inhibitors such as siRNA and shRNA against HDAC (e.g., HDAC1 siRNA Smartpool (registered trademark) (Millipore), HuSH 29mer shRNA Constructs against HDAC1 (OriGene))], MEK inhibitors (e.g., PD184352, PD98059, U0126, SL327, and PD0325901), Glycogen synthasekinase-3 inhibitors (e.g., Bio and CHIR99021), DNA methyltransferase inhibitors (e.g., 5-azacytidine), histone methyltransferase inhibitors (e.g., small molecule inhibitors such as BIX-01294, and nucleic acid-based expression inhibitors such as siRNA and shRNA against Suv39hl, Suv39h2, SetDBl, and G9a), L-channel calcium agonists (e.g., Bayk8644), butyric acid, TGFβ inhibitors or ALK5 inhibitors (e.g., LY364947, SB431542, 616453, and A-83-01), p53 inhibitors (e.g., siRNA and shRNA against p53), ARID3A inhibitors (e.g., siRNA and shRNA against ARID3A), miRNAs such as miR-291-3p, miR-294, miR-295, and mir-302, WntSignaling (e.g., soluble Wnt3a), neuropeptide Y, prostaglandins (e.g., prostaglandin E2 and prostaglandin J2), hTERT, SV40LT, UTF1, IRX6, GLISl, PITX2, DMRTBl, and the like. In the present specification, these factors for improving establishment efficiency are not particularly distinguished from reprogramming factors.

When the reprogramming factor is in the form of protein, it may be introduced into somatic cells by a technique such as lipofection, fusion with a cell-penetrating peptide (e.g., HIV-derived TAT and polyarginine), and/or microinjection, for example.

When the reprogramming factor is in the form of DNA, it can be introduced into somatic cells by a technique such as vectors (e.g., virus, plasmid, and artificial chromosome), lipofection, liposomes, and/or microinjection, for example. Examples of the viral vector include retroviral vectors and lentiviral vectors (those described in Cell, 126, pp. 663-676, 2006; Cell, 131, pp. 861-872, 2007; Science, 318, pp. 1917-1920, 2007), adenoviral vectors (Science, 322, 945-949, 2008), adeno-associated virus vectors, Sendai virus vectors (WO 2010/008054), and the like. Examples of the artificial chromosome vector include human artificial chromosome (HAC), yeast artificial chromosome (YAC), bacterial artificial chromosome (BAC, PAC), and the like. As the plasmid, a plasmid for mammalian cells may be used (Science, 322:949-953, 2008). The vector can harbor a regulatory sequence such as a promoter, an enhancer, a ribosome binding sequence, a terminator, and/or a polyadenylation site, for enabling expression of the nuclear reprogramming substance. The vector can also harbor, as needed, a selection marker sequence such as drug resistance gene (e.g., kanamycin resistance gene, ampicillin resistance gene, and/or puromycin resistance gene), thymidine kinase gene, and/or diphtheria toxin gene, and/or a reporter gene sequence such as green fluorescent protein (GFP), β glucuronidase (GUS), and/or FLAG, for example. Furthermore, in order that the gene encoding the reprogramming factor, or a combination of the gene encoding the reprogramming factor and a promoter to which the gene is to bind, can be cleaved off after introduction of the vector into somatic cells, the vector may have a LoxP sequence before and after these targets.

When the reprogramming factor is in the form of RNA, it may be introduced into somatic cells by a technique such as lipofection and/or microinjection, for example. For inhibiting degradation, 5-methylcytidine and pseudouridine (TriLink Biotechnologies) may be introduced into the RNA (Warren L, (2010) Cell Stem Cell. 7:618-630).

Examples of the culture medium for inducing iPS cells include DMEM, DMEM/F12, and DME culture media containing 10 to 15% FBS (these culture media can further contain LIF, penicillin/streptomycin, puromycin, L-glutamine, non-essential amino acids, β-mercaptoethanol, and/or the like, as appropriate), as well as commercially available culture media [e.g., culture media for mouse ES cell culture (TX-WES culture medium, Thrombo-X), culture media for primate ES cell culture (culture medium for primate ES/iPS cells, ReproCELL), and serum-free media (mTeSR, Stemcell Technology)].

As the method for culturing, the following method may be mentioned, for example. Firstly, at 37°C, in the presence of 5% CO₂, in a DMEM or DMEM/F12 culture medium containing 10% FBS, somatic cells are brought into contact with the reprogramming factor and incubated for about 4 to 7 days. Subsequently, the somatic cells thus incubated are plated over feeder cells (e.g., mitomycin C-treated STO cells and/or SNL cells), and after about 10 days following the contact of the somatic cells with the reprogramming factor, the somatic cells are incubated in a bFGF-containing culture medium dedicated for primate ES cell culture. After a lapse of at least about 30 to 45 days from the contact, iPS-like colonies can be formed.

Alternatively, at 37°C, in the presence of 5% CO₂, on feeder cells (e.g., mitomycin C-treated STO cells and/or SNL cells), culturing may be carried out in a DMEM culture medium containing 10% FBS (which can further contain LIF, penicillin/streptomycin, puromycin, L-glutamine, non-essential amino acids, β-mercaptoethanol, and/or the like, as appropriate), and at least about 25 to 30 days later, ES-like colonies can be formed. Instead of feeder cells, it may be desirable to use the somatic cells to be reprogrammed (Takahashi K, et al. (2009), PLoS One. 4:e8067, or WO2010/137746) or an extracellular matrix (e.g., Laminin-5 (WO2009/123349) and/or Matrigel (BD)).

In addition to the above-mentioned methods, a culture method using a serum-free medium may also be mentioned (Sun N, et al. (2009), Proc Natl Acad Sci U S A. 106:15720-15725). For enhancing the establishment efficiency, low-oxygen conditions (with an oxygen concentration from 0.1% to 15%) may be adopted for establishing iPS cells (Yoshida Y, et al. (2009), Cell Stem Cell. 5:237-241, or WO2010/013845).

During the above-described culturing, from the second day following the start of culturing, the culture medium is replaced by a fresh culture medium once a day. The number of somatic cells used in nuclear reprogramming is not limited, and it is within the range of about 5×10³ to about 5×10⁶ cells per 100 cm² of the culture dish.

iPS cells can be selected based on the shape of the formed colony. When a drug resistance gene expressable together with a gene (e.g., Oct3/4, Nanog) that is supposed to be expressed in the somatic cells once reprogrammed is introduced as a marker gene, culturing can be carried out in a culture medium (a selection culture medium) containing the target drug to select established iPS cells. In the case when a fluorescent protein gene is used as a marker gene, examination under a fluorescence microscope can be carried out to select iPS cells, or in the case of a luminescent enzyme gene, a luminescent substrate can be added, or in the case of a chromogenic enzyme gene, a chromogenic substrate can be added.

The term "somatic cells" used in the present specification refers to any animal cells (preferably cells of mammals including humans) except for germ-line cells such as eggs, oocytes, and ES cells and totipotent cells. The somatic cells encompass, without limitation, fetal (baby) somatic cells, newborn (baby) somatic cells, and healthy or diseased matured somatic cells, as well as primary cultured cells, subcultured cells, and established cell lines. In an aspect of the present embodiment, the somatic cells are preferably matured healthy somatic cells (somatic cells derived from a healthy subject). Specific examples of the somatic cells include: (1) tissue stem cells (somatic stem cells) such as neural stem cells, hematopoietic stem cells, mesenchymal stem cells, and dental pulp stem cells; (2) tissue progenitor cells; (3) differentiated cells such as lymphocytes, epithelial cells, endothelial cells, muscle cells, fibroblasts (e.g., skin cells), hair cells, hepatocytes, gastric mucosal cells, enterocytes, splenocytes, pancreatic cells (e.g., exocrine pancreatic cells), brain cells, lung cells, kidney cells, and adipocytes; and the like.

When using iPS cells as a material of cells for transplantation, it is desirable to use somatic cells that have the same or substantially the same HLA genotype as that of the recipient, from the viewpoint of avoiding rejection. Herein, "substantially the same" means that the HLA genotype matches to the extent that immune reactions against transplanted cells can be inhibited with an immunosuppressant, and, for example, the somatic cells may have an HLA type with three matched gene loci (HLA-A, HLA-B, and HLA-DR) or four matched gene loci (those three plus HLA-C).

### (E) ES Cells Derived from Cloned Embryo Obtained by Nuclear Transfer

ES cells derived from a cloned embryo obtained by nuclear transfer (ntES cells) are ES cells derived from a cloned embryo prepared by a nuclear transfer technique, and have almost the same properties as ES cells derived from a fertilized egg (T. Wakayama et al. (2001), Science, 292:740-743; S. Wakayama et al. (2005), Biol. Reprod., 72:932-936; J. Byrne et al. (2007), Nature, 450:497-502). More specifically, ntES (nuclear transfer ES) cells are ES cells established from the inner cell mass of a blastocyst derived from a cloned embryo obtained by replacing the nucleus of an unfertilized egg by the nucleus of a somatic cell. For preparation of ntES cells, a combination of a nuclear transfer technique (J. B. Cibelli et al. (1998), Nature Biotechnol., 16:642-646) and an ES cell preparation technique is used (Sayaka Wakayama et al (2008), Jikken Igaku (Experimental Medicine), vol. 26, No. 5 (Extra Edition), p. 47-52). In nuclear transfer, the nucleus of a somatic cell can be injected into an enucleated mammalian unfertilized egg, which can then be incubated for several hours for reprogramming.

### (F) Multilineage-Differentiating Stress Enduring Cells

Multilineage-differentiating stress enduring cells (Muse cells) are pluripotent stem cells produced by a method described in WO2011/007900. More specifically, Muse cells are pluripotent cells that are obtained by trypsin treatment of fibroblasts or bone marrow stromal cells performed for a prolonged period of time, preferably trypsin treatment performed for 8 hours or 16 hours, followed by suspension culture, and are positive for SSEA-3 and CD105.

In the present embodiment, preferable pluripotent stem cells are human iPS cells. In an aspect of the present embodiment, the human iPS cells may be human iPS cells derived from matured healthy somatic cells (human iPS cells derived from a healthy subject), or may be human iPS cells derived from diseased somatic cells (disease-specific iPS cells). In another aspect of the present embodiment, the human iPS cells are preferably human iPS cells derived from a healthy subject.

The method for inducing cardiomyocytes from pluripotent stem cells is not particularly limited as long as it is a known method, and examples thereof include (1) a method implemented in the absence of feeder cells and (2) a method implemented in the presence of feeder cells.

In the present embodiment, examples of (1) the method for inducing cardiomyocytes from pluripotent stem cells in the absence of feeder cells include a method comprising (i) a step to incubate induced pluripotent stem cells in a medium containing Activin A and, after the step (i), (ii) a step to further incubate the cells in a medium containing BMP4 and bFGF.

### (1) Method for Inducing Cardiomyocytes from Pluripotent Stem Cells in Absence of Feeder Cells

### (i) Step to Incubate in Medium Containing Activin A

In this step, pluripotent stem cells may be isolated by a freely-selected method, followed by suspension culture or adherent culture on a coated culture dish. Preferred is adherent culture. Herein, the method for isolation may be a mechanical isolation method, or an isolation method using an isolation solution (e.g., Accutase (trade mark) and Accumax (trade mark)) having both protease activity and collagenase activity or an isolation liquid having only collagenase activity. Preferred is a method involving dissociating the cells with use of an isolation liquid having only collagenase activity and mechanically isolating them from each other. Preferably, the pluripotent stem cells used here are colonies formed by incubation to about 80% confluency in the dish.

The suspension culture refers to incubating cells in a state where they are not adhered to the culture dish. The suspension culture is not particularly limited, and it can be carried out by using a culture dish without any artificial treatment (e.g., coating treatment with extracellular matrix and/or the like) performed for the purpose of enhancing cell adhesion, or a culture dish with treatment (e.g., coating treatment with polyhydroxyethyl methacrylate (poly-HEMA)) performed for the purpose of artificially reducing adhesion.

The adherent culture refers to a method of culturing in a freely-selected medium in a coated culture dish. Examples of the coating agent include Matrigel (BD), collagen, gelatin, laminin, heparan sulfate proteoglycan, and entactin, as well as combinations of these. A preferable coating agent is Matrigel. More preferred is a Matrigel sandwich adherent culture that involves making induced pluripotent stem cells adhere to a Matrigel-coated culture dish and then adding Matrigel to the medium to fully coat the pluripotent stem cells with Matrigel.

The medium in the step (i) can be prepared by using a medium for use in animal cell culture as a basal medium and adding Activin A to the basal medium.

The basal medium encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is RPMI 1640 medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. A preferable basal medium in the step (i) may be RPMI medium containing L-glutamine and B27 supplement.

The medium in the step (i) may contain not only Activin A but also one or more growth factors selected from the group consisting of Wnt1, Wnt3, Wnt3a, Wnt4, Wnt7a, TGF-β, Nodal, BMP2, BMP4, BMP6, BMP7, GDF, bFGF, and VEGF added to the basal medium. A preferable growth factor is Wnt3a.

The concentration of Activin A in the medium is, without limitation, 10 ng/mL, 25 ng/mL, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL, 175 ng/mL, or 200 ng/mL, for example. Preferably, the concentration of Activin A in the medium is 100 ng/mL. In an aspect of the present embodiment, the concentration of Activin A in the medium may be from 10 ng/mL to 200 ng/mL.

The concentration of Wnt3a in the medium is, without limitation, 10 ng/mL, 25 ng/mL, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL, 175 ng/mL, or 200 ng/mL, for example. Preferably, the concentration of Wnt3a in the medium is 100 ng/mL. In an aspect of the present embodiment, the concentration of Wnt3a in the medium may be from 10 ng/mL to 200 ng/mL.

The incubation temperature is, without limitation, approximately from 30 to 40°C, preferably about 37°C. Incubation is carried out in an atmosphere with CO₂-containing air. Preferably, the CO₂ concentration at this time is approximately from 2 to 5%. The incubation duration is from 1 day to 5 days, for example, and preferably 1 day.

### (ii) Step to Incubate in Medium Containing BMP and bFGF

In the step (ii), in the case where the upstream step was implemented by suspension culture, the resulting cell population may be incubated, as it is, in a freely-selected medium in a coated culture dish. Examples of the coating agent include Matrigel (BD), collagen, gelatin, laminin, heparan sulfate proteoglycan, and entactin, as well as combinations of these. Preferred is Matrigel. In the case where the upstream step was implemented by adherent culture, incubation may be continued by changing the medium.

The medium used in the step (ii) can be prepared by using a medium for use in animal cell culture as a basal medium and adding BMP and bFGF to the basal medium.

As the basal medium, the same one used in the above-mentioned step (i) can be used.

The BMP used in the step (ii) is preferably a BMP belonging to the superfamily TGFβ, and examples thereof include BMP2, BMP4, and BMP7. A preferable BMP is BMP4.

The concentration of BMP4 in the medium is, without limitation, 0.1 ng/mL, 0.5 ng/mL, 1 ng/mL, 2.5 ng/mL, 5 ng/mL, 6 ng/mL, 7 ng/mL, 8 ng/mL, 9 ng/mL, 10 ng/mL, 11 ng/mL, 12 ng/mL, 13 ng/mL, 14 ng/mL, 15 ng/mL, 17.5 ng/mL, 20 ng/mL, 30 ng/mL, 40 ng/mL, or 50 ng/mL, for example. Preferably, the concentration of BMP4 in the medium is 10 ng/mL. In an aspect of the present embodiment, the concentration of BMP4 in the medium may be from 0.1 ng/mL to 50 ng/mL.

The concentration of bFGF in the medium is, without limitation, 0.1 ng/mL, 0.5 ng/mL, 1 ng/mL, 2.5 ng/mL, 5 ng/mL, 6 ng/mL, 7 ng/mL, 8 ng/mL, 9 ng/mL, 10 ng/mL, 11 ng/mL, 12 ng/mL, 13 ng/mL, 14 ng/mL, 15 ng/mL, 17.5 ng/mL, 20 ng/mL, 30 ng/mL, 40 ng/mL, or 50 ng/mL, for example. Preferably, the concentration of bFGF in the medium is 10 ng/mL. In an aspect of the present embodiment, the concentration of bFGF in the medium may be from 0.1 ng/mL to 50 ng/mL.

The incubation temperature is, without limitation, approximately from 30 to 40°C, preferably about 37°C. Incubation is carried out in an atmosphere with CO₂-containing air. Preferably, the CO₂ concentration at this time is approximately from 2 to 5%. The incubation duration is from 1 day to 10 days, for example, and preferably 4 days.

### (2) Method Implemented in Presence of Feeder Cells

In the present embodiment, as 2) the method for inducing cardiomyocytes from pluripotent stem cells in the presence of feeder cells, a method of co-incubating pluripotent stem cells or Flk1-positive cells derived from pluripotent stem cells with OP9 cells (Nishikawa, S. I. et al, Development 125, 1747-1757 (1998)) or END-2 cells (Mummery C, et al, Circulation. 107:2733-40 (2003)) may be mentioned.

The medium in co-culture with feeder cells can be prepared by using a medium for use in animal cell culture as a basal medium and adding additives thereto as appropriate.

The basal medium encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is RPMI 1640 medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. Preferable basal media may be αMEM medium containing 10% FBS, αMEM medium containing 10% FBS, and DMEM medium containing 10% FBS.

As an additive to add to the basal medium in co-culture with feeder cells, cyclosporine A, activin A, and BMP4 may be added in an amount from 1 to 3 µg/mL.

The incubation temperature is, without limitation, approximately from 30 to 40°C, preferably about 37°C. Incubation is carried out in an atmosphere with CO₂-containing air. Preferably, the CO₂ concentration at this time is approximately from 2 to 5%. The incubation duration, which is the number of days required for expression of myocardial troponin and/or αMHC, is from 10 days to 20 days, for example.

Preferable conditions may be as follows: after incubation in αMEM medium containing 10% FBS for 4 days, Flk1-positive cells are isolated and then co-cultured with OP9 cells for 6 days in αMEM medium containing 3 µg/mL cyclosporine A and 10% FBS; or co-culture is carried out with END-2 cells for 16 days in DMEM medium containing 10% FBS.

The cardiomyocytes thus obtained may be further incubated in a basal medium supplemented with VEGF, to be used for preparing an engineered cardiac tissue according to the present embodiment. As needed, a Wnt signal transduction pathway inhibitor (e.g., XAV939, IWP4) may be added to the basal medium.

The basal medium to which VEGF is added encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is RPMI 1640 medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. A preferable basal medium in this step may be RPMI medium containing L-glutamine and B27 supplement.

The concentration of VEGF in the medium may be within the range of 10 ng/mL to 500 ng/mL, or 25 ng/mL to 300 ng/mL, or 40 ng/mL to 200 ng/mL, or 50 ng/mL to 100 ng/mL, or 60 ng/mL to 90 ng/mL, or 65 ng/mL to 85 ng/mL, for example. Preferably, the concentration of VEGF in the medium is from 25 ng/mL to 75 ng/mL. The concentration of VEGF in the medium may be, without limitation, 10 ng/mL, 25 ng/mL, 50 ng/mL, 55 ng/mL, 60 ng/mL, 65 ng/mL, 70 ng/mL, 75 ng/mL, 80 ng/mL, 85 ng/mL, 90 ng/mL, 95 ng/mL, 100 ng/mL, 110 ng/mL, 120 ng/mL, 130 ng/mL, 140 ng/mL, 150 ng/mL, or 200 ng/mL. Preferably, the concentration of VEGF in the medium is 50 ng/mL.

The incubation temperature is, without limitation, approximately from 30 to 40°C, preferably about 37°C. Incubation is carried out in an atmosphere with CO₂-containing air. Preferably, the CO₂ concentration at this time is approximately from 2 to 5%. The incubation duration is from 4 days to 20 days, for example, and preferably 10 days.

In the present embodiment, the content ratio of the immature cardiomyocytes to all the cells contained in the sample may be from 10% to 80%, or from 20% to 70%. The number of the immature cardiomyocytes can be counted by flow cytometry using TNNI1 as a marker molecule, for example.

### (Vascular Mural Cells)

In the present embodiment, the sample preferably further contains vascular mural cells. "Vascular mural cells" (hereinafter also simply referred to as "mural cells") means cells that express smooth muscle actin (SMA) and/or PDGFRB. SMA of humans may be NCBI Accession Number NM_001141945, and that of mice may be NM_007392. PDGFRB of humans may be NCBI Accession Number NM_002609, and that of mice may be NM_001146268.

The method for inducing mural cells from pluripotent stem cells is not particularly limited as long as it is a known method, and examples thereof include a method comprising (I) a step to incubate pluripotent stem cells in a medium containing Activin A, (II) a step to incubate the cells obtained in the step (I) in a medium containing BMP and bFGF, and (III) a step to incubate the cells obtained in the step (II) in a VEGF-free medium.

### (I) Step to Incubate in Medium Containing Activin A

In this step, pluripotent stem cells may be isolated by a freely-selected method, followed by suspension culture or adherent culture on a coated culture dish. Preferred is adherent culture. Herein, the method for isolation may be a mechanical isolation method, or an isolation method using an isolation solution (e.g., Accutase (trade mark) and Accumax (trade mark)) having both protease activity and collagenase activity or an isolation liquid having only collagenase activity. Preferred is a method involving dissociating the cells with use of an isolation liquid having only collagenase activity and mechanically isolating them from each other. Preferably, the pluripotent stem cells used here are colonies formed by incubation to 80% confluency in the dish.

The suspension culture refers to incubating cells in a state where they are not adhered to the culture dish. The suspension culture is not particularly limited, and it can be carried out by using a culture dish without any artificial treatment (e.g., coating treatment with extracellular matrix and/or the like) performed for the purpose of enhancing cell adhesion, or a culture dish with treatment (e.g., coating treatment with polyhydroxyethyl methacrylate (poly-HEMA)) performed for the purpose of artificially reducing adhesion.

The adherent culture refers to a method of culturing in a freely-selected medium in a coated culture dish. Examples of the coating agent include Matrigel (BD), collagen, gelatin, laminin, heparan sulfate proteoglycan, and entactin, as well as combinations of these. A preferable coating agent is Matrigel. More preferably, the adherent culture is a Matrigel sandwich adherent culture that involves making induced pluripotent stem cells adhere to a Matrigel-coated culture dish and then adding Matrigel to the medium to fully coat the pluripotent stem cells with Matrigel.

The medium in the step (I) can be prepared by using a medium for use in animal cell culture as a basal medium and adding Activin A to the basal medium.

The basal medium encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is RPMI 1640 medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. A preferable basal medium in the step (I) may be RPMI medium containing L-glutamine and B27 supplement.

t The medium in the step (I) may contain not only Activin A but also one or more growth factors selected from the group consisting of Wnt1, Wnt3, Wnt3a, Wnt4, Wnt7a, TGF-β, Nodal, BMP2, BMP4, BMP6, BMP7, GDF, bFGF, and VEGF added to the basal medium. A preferable growth factor is Wnt3a.

The concentration of Activin A in the medium is, without limitation, 10 ng/mL, 25 ng/mL, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL, 175 ng/mL, or 200 ng/mL, for example. Preferably, the concentration of Activin A in the medium is 100 ng/mL. In an aspect of the present embodiment, the concentration of Activin A in the medium may be from 10 ng/mL to 200 ng/mL.

The concentration of Wnt3a in the medium is, without limitation, 10 ng/mL, 25 ng/mL, 50 ng/mL, 60 ng/mL, 70 ng/mL, 80 ng/mL, 90 ng/mL, 100 ng/mL, 110 ng/mL, 120 ng/mL, 130 ng/mL, 140 ng/mL, 150 ng/mL, 175 ng/mL, or 200 ng/mL, for example. Preferably, the concentration of Wnt3a in the medium is 100 ng/mL. In an aspect of the present embodiment, the concentration of Wnt3a in the medium may be from 10 ng/mL to 200 ng/mL.

The incubation temperature is, without limitation, approximately from 30 to 40°C, preferably about 37°C. Incubation is carried out in an atmosphere with CO₂-containing air. Preferably, the CO₂ concentration at this time is approximately from 2 to 5%. The incubation duration is from 1 day to 5 days, for example, and preferably 1 day.

### (II) Step to Incubate in Medium Containing BMP and bFGF

In the step (II), in the case where the upstream step was implemented by suspension culture, the resulting cell population may be incubated, as it is, in a freely-selected medium in a coated culture dish. Examples of the coating agent include Matrigel (BD), collagen, gelatin, laminin, heparan sulfate proteoglycan, and entactin, as well as combinations of these. A preferable coating agent is Matrigel. In the case where the upstream step was implemented by adherent culture, incubation may be continued by changing the medium.

The medium used in the step (II) can be prepared by using a medium for use in animal cell culture as a basal medium and adding BMP and bFGF to the basal medium.

As the basal medium, the same one used in the above-mentioned step (I) can be used.

The BMP used in the step (II) is preferably a BMP belonging to the superfamily TGFβ, and examples thereof include BMP2, BMP4, and BMP7. A preferable BMP is BMP4.

The concentration of BMP4 in the medium is, without limitation, 0.1 ng/mL, 0.5 ng/mL, 1 ng/mL, 2.5 ng/mL, 5 ng/mL, 6 ng/mL, 7 ng/mL, 8 ng/mL, 9 ng/mL, 10 ng/mL, 11 ng/mL, 12 ng/mL, 13 ng/mL, 14 ng/mL, 15 ng/mL, 17.5 ng/mL, 20 ng/mL, 30 ng/mL, 40 ng/mL, or 50 ng/mL, for example. Preferably, the concentration of BMP4 in the medium is 10 ng/mL. In an aspect of the present embodiment, the concentration of BMP4 in the medium may be from 0.1 ng/mL to 50 ng/mL.

The concentration of bFGF in the medium is, without limitation, 0.1 ng/mL, 0.5 ng/mL, 1 ng/mL, 2.5 ng/mL, 5 ng/mL, 6 ng/mL, 7 ng/mL, 8 ng/mL, 9 ng/mL, 10 ng/mL, 11 ng/mL, 12 ng/mL, 13 ng/mL, 14 ng/mL, 15 ng/mL, 17.5 ng/mL, 20 ng/mL, 30 ng/mL, 40 ng/mL, or 50 ng/mL, for example. Preferably, the concentration of bFGF in the medium is 10 ng/mL. In an aspect of the present embodiment, the concentration of bFGF in the medium may be from 0.1 ng/mL to 50 ng/mL.

The incubation temperature is, without limitation, approximately from 30 to 40°C, preferably about 37°C. Incubation is carried out in an atmosphere with CO₂-containing air. Preferably, the CO₂ concentration at this time is approximately from 2 to 5%. The incubation duration is from 1 day to 10 days, for example, and preferably 2 days.

### (III) Step to Incubate in VEGF-Free Medium

The cells obtained in the step (II) are incubated in a basal medium, which is a VEGF-free medium for use in animal cell culture. The VEGF-free medium may be a medium that substantially does not contain VEGF, and, for example, the VEGF concentration is less than 1 ng/mL, preferably less than 0.1 ng/mL, more preferably 0.

The basal medium used in the step (III) encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is RPMI 1640 medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. A preferable basal medium used in the step (III) may be RPMI medium containing 10% FBS.

In the present embodiment, the content ratio of the vascular mural cells to all the cells contained in the sample may be from 5% to 80%, or from 10% to 70%. The number of the vascular mural cells can be counted by flow cytometry using SMA or PDGFRB as a marker molecule, for example.

### (Vascular Endothelial Cells)

In the present embodiment, the sample preferably further contains vascular endothelial cells. With vascular endothelial cells thus contained, formation of vascular networks is facilitated at the time when hydrostatic pressure is applied to the sample (e.g., ECT). "Vascular endothelial cells" (hereinafter also simply referred to as "endothelial cells") means cells that express one of PE-CAM, VE-cadherin, and von Willebrand factor (vWF). PE-CAM of humans may be NCBI Accession Number NM_000442, and that of mice may be NM_001032378. VE-cadherin of humans may be NCBI Accession Number NM_001795, and that of mice may be NM_009868. vWF of humans may be NCBI Accession Number NM_000552, and that of mice may be NM_011708.

The method for inducing endothelial cells from pluripotent stem cells is not particularly limited as long as it is a known method, and examples thereof include a method comprising (a) a step to incubate pluripotent stem cells in a medium containing Activin A and Wnt3a, (b) a step to incubate the cells obtained in the step (a) in a medium containing BMP and bFGF, and (c) a step to incubate the cells obtained in the step (b) in a medium containing VEGF.

The steps (a), (b), and (c) may be the same as those in the above-described method for inducing cardiomyocytes. Hence, in the present embodiment, it is possible to use a method for simultaneously inducing cardiomyocytes and endothelial cells comprising the steps (a), (b), and (c). As a specific procedure, a method (CM+EC protocol) described in Examples below may be mentioned, for example.

In the present embodiment, cAMP for inducing endothelial cells may be further added in the step (c). The concentration of cAMP is more than 0.5 mM and less than 2 mM, for example, and it is, without limitation, 0.6 mM, 0.7 mM, 0.8 mM, 0.9 mM, 1 mM, 1.1 mM, 1.2 mM, 1.3 mM, 1.4 mM, 1.5 mM, 1.6 mM, 1.7 mM, 1.8 mM, or 1.9 mM. Preferably, it is 1 mM. The duration for adding cAMP is not particularly limited, and it is preferably from 1 day to 5 days, particularly preferably 3 days.

In the present embodiment, the content ratio of the vascular endothelial cells to all the cells contained in the sample may be from 5% to 80%, or from 10% to 70%. The number of the vascular endothelial cells can be counted by flow cytometry using PE-CAM, VE-cadherin, or vWF as a marker molecule, for example.

In the present embodiment, the sample is preferably an engineered cardiac tissue (ECT) derived from iPS cells. "Engineered cardiac tissue" means a man-made three-dimensional cardiac tissue structure, or a man-made three-dimensional structure similar to the structure of cardiac tissue. The "engineered cardiac tissue derived from iPS cells" means an engineered cardiac tissue that at least contains cardiomyocytes differentiated from iPS cells. In an aspect of the present embodiment, the "engineered cardiac tissue derived from iPS cells" can also be understood as an engineered cardiac tissue that has a genome derived from the same origin as that of the iPS cells used in the induced differentiation. Preferably, the engineered cardiac tissue further contains either vascular endothelial cells or vascular mural cells, or both. Preferably, the engineered cardiac tissue is a structure that spontaneously contracts and expands in a repeated manner.

The method of producing an engineered cardiac tissue is not particularly limited and may be a known method. For example, it is possible to mix the above-mentioned cardiomyocytes, endothelial cells, and mural cells and incubate them in the presence of an extracellular matrix to form a three-dimensional structure so as to obtain an engineered cardiac tissue. The mural cells are preferably mixed in a ratio less than 30%; more preferably the mural cells are mixed in a ratio within the range of 5% to 25%; further preferably the mural cells are mixed in a ratio from 5% to 20%; most preferably the mural cells are mixed in a ratio from 10% to 20%.

In the present embodiment, the extracellular matrix means a protein or proteins that are secreted to the outside of cells, and examples of the extracellular matrix include collagen, gelatin, laminin, heparan sulfate proteoglycan, entactin, fragments of these, and combinations of these. The extracellular matrix used for formation of the engineered cardiac tissue is not particularly limited; it is an extracellular matrix containing at least type I collagen, more preferably Matrigel (available from BD) containing type I collagen.

The medium used for formation of the engineered cardiac tissue encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is DMEM medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. A preferable medium to be used for formation of the engineered cardiac tissue may be DMEM medium containing 20% FBS.

Formation of the engineered cardiac tissue can be carried out by mixing a medium in which the above-mentioned cell mixture is suspended, with an extracellular matrix, placing the resultant in a vessel having a desired shape, and leaving it for a certain period of time. From the viewpoint of easy handling of the engineered cardiac tissue, it is desirable that the part of the vessel to come into contact with the cell mixture is covered with a silicon film coated with type I collagen. A vessel of this type is available from Flexcell International. From the viewpoint of easy handling of the engineered cardiac tissue thus formed, the shape of the vessel is preferably columnar, desirably cylindrical.

The engineered cardiac tissue thus formed can be stored in a freely-selected medium, and the medium encompasses IMDM medium, Medium 199 medium, Eagle's Minimum Essential Medium (EMEM), αMEM medium, Dulbecco's modified Eagle's Medium (DMEM), Ham's F12 medium, RPMI 1640 medium, Fischer's medium, and a mixture of these media, for example. Preferred is DMEM medium. The basal medium may contain serum, or may be free of serum. As needed, the basal medium may contain one or more serum replacements such as, for example, albumin, transferrin, Knockout Serum Replacement (KSR) (serum replacement for replacing FBS in ES cell culture), N2 supplement (Invitrogen), B27 supplement (Invitrogen), fatty acids, insulin, collagen precursors, trace elements, 2-mercaptoethanol, 3'-thiolglycerol, and the like. Moreover, the basal medium may also contain one or more substances such as lipids, amino acids, L-glutamine, Glutamax (Invitrogen), non-essential amino acids, vitamins, antibiotics, antioxidants, pyruvic acid, buffers, and inorganic salts. A preferable medium for incubating the engineered cardiac tissue may be αMEM medium containing 10% FBS, 2-mercaptoethanol, and antibiotics.

### <Pressure-Applying Step>

In this step, hydrostatic pressure is continuously or intermittently applied to the sample. In the present embodiment, "hydrostatic pressure" means a pressure applied by static water or static aqueous solution (e.g., medium). In the present embodiment, the expression "hydrostatic pressure is continuously applied" means that application of hydrostatic pressure in the below-described cycle is maintained for a certain period of time. The expression "hydrostatic pressure is intermittently applied" means that application of hydrostatic pressure in the below-described cycle and a pause of the cycle are repeated in an alternating manner.

In the present embodiment, the method for applying hydrostatic pressure to the sample is not particularly limited as long as the cells contained in the sample can survive in the environment. For example, hydrostatic pressure may be applied with the use of a MechanoCulture TR stimulation apparatus (MCTR, manufactured by CellScale) mentioned in Examples. Herein, the "environment in which the cells can survive" may be, for example, an environment in which the cells are in contact with the medium used for incubating the cells.

In the present embodiment, the applied pressure of the hydrostatic pressure applied in the pressure-applying step (the applied pressure of the hydrostatic pressure applied in a compress duration described below) is preferably from 10 kPa to 100 kPa, more preferably from 25 kPa to 75 kPa.

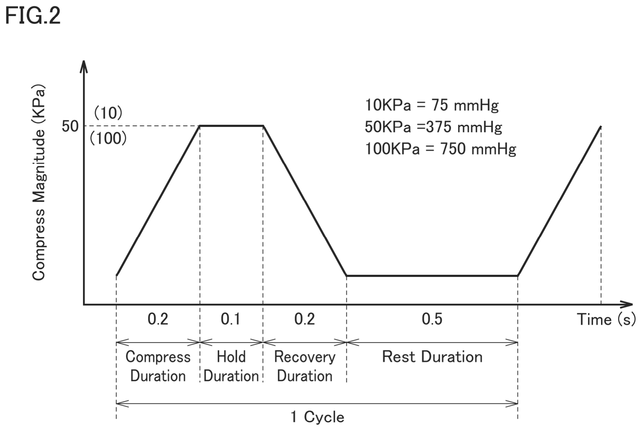

In the present embodiment, the "cycle" for applying hydrostatic pressure means a unit consisting of Compress duration → Hold duration → Recovery duration → Rest duration, and this unit is implemented repeatedly (see Fig. 2). During the compress duration, the hydrostatic pressure rises from "the pre-application pressure" to reach the above-mentioned applied pressure, and during the hold duration, the above-mentioned applied pressure is maintained. Then, during the recovery duration, the hydrostatic pressure drops from the above-mentioned applied pressure to reach "the pre-application pressure", and during the rest duration, "the pre-application pressure" is maintained.

In the present embodiment, the manner the hydrostatic pressure rises during the compress duration is not particularly limited as long as the hydrostatic pressure reaches the above-mentioned applied pressure, and the hydrostatic pressure may rise either linearly or non-linearly. The compress duration preferably lasts for 0.05 seconds to 0.5 seconds, more preferably for 0.1 seconds to 0.3 seconds.

In the present embodiment, the hold duration preferably lasts for 0.025 seconds to 0.25 seconds, more preferably for 0.05 seconds to 0.15 seconds.

In the present embodiment, the manner the hydrostatic pressure drops during the recovery duration is not particularly limited as long as the hydrostatic pressure drops to reach the pre-application pressure, and the hydrostatic pressure may drop either linearly or non-linearly. The recovery duration preferably lasts for 0.05 seconds to 0.5 seconds, more preferably for 0.1 seconds to 0.3 seconds.

In the present embodiment, the rest duration preferably lasts for 0.25 seconds to 0.75 seconds, more preferably for 0.4 seconds to 0.6 seconds.

In the present embodiment, the length of time of the above-mentioned cycle is preferably from 0.25 seconds to 4 seconds, more preferably from 0.5 seconds to 2 seconds. The length of time of the above-mentioned cycle can also be understood as a total of the compress duration, the hold duration, the recovery duration, and the rest duration in one cycle.

In the present embodiment, in the pressure-applying step, it is preferable that the hydrostatic pressure be applied intermittently at a frequency from 1 hour to 12 hours per day, and more preferably, it is applied intermittently at a frequency from 1 hour to 6 hours per day. In an aspect of the present embodiment, the expression "hydrostatic pressure is applied intermittently at a frequency of 1 hour per day" can also be understood as that application of hydrostatic pressure in the above-mentioned cycle is maintained for 1 hour and then no application of hydrostatic pressure lasts for 23 hours.

In the present embodiment, the pressure-applying step is preferably implemented for a period from 3 days to 8 days, more preferably for a period from 3 days to 5 days.

Conventionally, stimulation by hydrostatic pressure application is performed in tendon and bone researches but not in cardiac tissue. Moreover, as physical stimulation of cardiac tissue, electric stimulation and stretching-direction stimulation are performed but compressing-direction stimulation such as hydrostatic pressure is not performed. The inventors of the present invention have found that application of a certain amount of hydrostatic pressure to a sample containing immature cardiomyocytes can efficiently mature the cardiomyocytes, and thus, completed the present invention. It should be noted that a living heart beats constantly, even while it is developing. While the heart is beating, cardiomyocytes are maturing under the stimulation applied by beating. Because of this, it is considered that continuous application of pressure is also preferable when artificially applying hydrostatic pressure. However, surprisingly, the inventors of the present invention have found that in the case of artificial application of hydrostatic pressure, maturation effectively proceeds when the pressure is applied intermittently. The inventors of the present invention have also found that maturation proceeds better when the applied hydrostatic pressure is higher than normal blood pressure (10 kPa).

In the present embodiment, "matured cardiomyocytes" means cells that have matured from the above-mentioned immature cardiomyocytes due to stimulation applied by hydrostatic pressure and are positive for TNNI3. Other examples of markers for matured cardiomyocytes include MYH7, NPPA, and the like.

### <<Method of Producing Matured Cardiomyocytes>>

In the present embodiment, the above-mentioned method of maturing cardiomyocytes can also be understood as a method of producing matured cardiomyocytes.

That is, a method of producing matured cardiomyocytes according to the present embodiment comprises:
a preparation step to prepare a sample containing immature cardiomyocytes; and
a pressure-applying step to apply hydrostatic pressure continuously or intermittently to the sample.

Details of the steps are the same as in the description for the above-mentioned method of maturing cardiomyocytes.

### <<Engineered Cardiac Tissue>>

The engineered cardiac tissue according to the present embodiment is an engineered cardiac tissue containing cardiomyocytes, wherein
the cardiomyocytes include TNNI3-positive cells.

In an aspect of the present embodiment, the engineered cardiac tissue can also be understood as an engineered cardiac tissue obtained by maturing immature cardiomyocytes contained in a precursor of the engineered cardiac tissue by the method of maturing cardiomyocytes according to the present embodiment.

In the present embodiment, the content ratio of the cardiomyocytes to all the cells contained in the engineered cardiac tissue may be from 10% to 80%, or from 20% to 70%. The number of the cardiomyocytes can be counted by flow cytometry using cTnT as a marker molecule, for example.

In an aspect of the present embodiment, the content ratio of TNNI3-positive cells to all the cells contained in the engineered cardiac tissue may be from 5% to 70%, or from 10% to 50%. The number of TNNI3-positive cells can be counted by flow cytometry using TNNI3 as a marker molecule, for example.

In an aspect of the present embodiment, the amount of expression of TNNI3 in the TNNI3-positive cells relative to the amount of expression of TNNI3 in immature cardiomyocytes is preferably from 100% to 1000%, more preferably from 200% to 800%. The amount of expression of TNNI3 can be estimated by quantitative PCR.

The engineered cardiac tissue may further contain vascular endothelial cells. With vascular endothelial cells thus further contained, the engineered cardiac tissue has excellent mechanical contractile ability. In the present embodiment, the content ratio of the vascular endothelial cells to all the cells contained in the engineered cardiac tissue may be from 5% to 80%, or from 10% to 70%. The number of the vascular endothelial cells can be counted by flow cytometry using PE-CAM, VE-cadherin, or vWF as a marker molecule, for example.

The engineered cardiac tissue may further contain vascular mural cells. With vascular mural cells thus further contained, the engineered cardiac tissue has excellent tissue strength. In the present embodiment, the content ratio of the vascular mural cells to all the cells contained in the engineered cardiac tissue may be from 5% to 80%, or from 10% to 70%. The number of the vascular mural cells can be counted by flow cytometry using SMA or PDGFRB as a marker molecule, for example.

In an aspect of the present embodiment, the engineered cardiac tissue may further contain an extracellular matrix. Specific examples of the extracellular matrix include those mentioned above.

### <Therapeutic Agent for Heart Diseases>

The present embodiment provides a therapeutic agent for heart diseases containing the above-mentioned engineered cardiac tissue. The heart disease to which the present embodiment can be applied is not particularly limited as long as it is a pathological condition with loss of cardiomyocytes and the like, and examples thereof include heart failure, ischemic heart disease, myocardial infarction, cardiomyopathy, myocarditis, hypertrophic cardiomyopathy, dilated-phase of hypertrophic cardiomyopathy, dilated cardiomyopathy, and the like.

The engineered cardiac tissue according to the present embodiment, after molded, may be directly sutured to a site of loss of cardiomyocytes, a site of infarction, a site of lesion, or a site of disorder, or may be transplanted to such sites with the use of a biological glue that is for use as adhesive for organs, such as fibrin glue.

The myocardial tissue for use in the heart disease treatment may be prepared as appropriate to be suitable for the size of the target area and/or the size of the body.

### [Examples]

In the following, a more detailed description will be given of the present invention by way of examples, but these examples are not intended to limit the scope of the present invention.

### <<Experiment Procedure>>

### <<Culturing and Induced Differentiation of Human iPS Cells>>

Maintenance culture of human iPS cells and subsequent induced differentiation were carried out by the following procedures. As human iPS cells, the following two cell lines were used.

Four-factor (Oct3/4, Sox2, Klf4, and c-Myc) cell line (human iPS cell line derived from a healthy subject): 201B6

Disease-specific iPS cell line: HPS1799

### (Maintenance Culture of Human iPS Cells)

Firstly, in StemFit AK02N medium (manufactured by Ajinomoto Co. Inc.; hereinafter also referred to as "AK02N medium"), expansion culture and maintenance culture of each type of the iPS cells were carried out. After the iPS cells reached confluency during incubation, TrypLE Select (manufactured by Thermo Fisher Scientific) was added to dissociate the iPS cells, and PBS (0.5 mM ethylenediaminetetraacetic acid) in the same amount (volume) as that of the added TrypLE Select was further added to suspend the iPS cells. Subsequently, the iPS cells in a state of single cells (5000 to 8000 cells/cm²) were passaged every 7 days. For passaging, AK02N medium supplemented with iMatrix-511 silk (manufactured by FUJIFILM Wako Pure Chemical Corporation) (final concentration, 0.125 µg/cm²) (uncoated laminin fragment) and ROCK inhibitor (Y-27632, in a final concentration of 10 µM) (manufactured by FUJIFILM Wako Pure Chemical Corporation) was used.

### (Common Upstream Step in Induced Differentiation of Cardiovascular Cells)

To begin with, a description will be given of an upstream step that is common among the CM+EC protocol, the CM protocol, and the MC protocol described below. Herein, cardiomyocytes (CMs), vascular endothelial cells (ECs), and vascular mural cells (MCs) may also be collectively referred to as "cardiovascular cells" (CV cells). Firstly, iPS cells in a state of single cells were plated in 360000 to 400000 cells/cm² in AK02N medium (containing Y-27632 in a final concentration of 10 µM) in a culture plate coated with Matrigel (diluted to 1:60). After the iPS cells reached confluency during incubation, and one day before induced differentiation, the iPS cells were coated with Matrigel (diluted with AK02N medium to 1:60). After a lapse of 1 day, the medium was changed from AK02N medium to RPMI+B27 medium (Day 0 of induced differentiation; d0), followed by incubation for 24 hours. The composition of the RPMI+B27 medium is as follows.
(Composition of RPMI+B27 Medium)
RPMI 1640 medium (manufactured by Thermo Fisher)
L-glutamine (final concentration, 2 mM) (manufactured by Thermo Fisher)
1×B27 supplement without insulin (manufactured by Thermo Fisher)
Activin A (final concentration, 100 ng/mL) (manufactured by R&D)
CHIR99021 (final concentration, 5 µM; as needed) (manufactured by Tocris Bioscience)

Subsequently, on Day 1 of induced differentiation (d1), bone morphogenetic protein 4 (BMP4, in a final concentration of 10 ng/mL) (manufactured by R&D) and basic fibroblast growth factor (bFGF, in a final concentration of 10 ng/mL) were added to the culture medium, followed by incubation for 4 days (incubation for 2 days in the MC protocol). At this time, the medium was not changed. Subsequent processes (downstream steps) will be described separately for the CM+EC protocol, the CM protocol, and the MC protocol.

### (Downstream Step of CM+EC Protocol)

For differentiation into CMs and ECs, the medium was changed to RPMI 1640 medium on Day 5 of induced differentiation (d5). The RPMI 1640 medium used here contained vascular endothelial growth factor (VEGF) 165 (final concentration, 50 ng/ml) (manufactured by FUJIFILM Wako Pure Chemical Corporation), and, as needed, IWP4 (final concentration, 2.5 µM) (manufactured by Stemgent) and XAV939 (final concentration, 5 µM) (manufactured by Merck). Subsequently, every other day, the medium was changed to RPMI 1640 medium (free of insulin) supplemented with VEGF165 (final concentration, 50 ng/ml). During the time period from Day 11 of induced differentiation (d11) to Day 15 (d15), beating cells started to appear.

### (Downstream Step of CM Protocol)

For differentiation into CMs, the medium was changed to RPMI+B27 medium (containing insulin) on Day 5 of induced differentiation (d5). The RPMI+B27 medium used here contained, as needed, IWP4 (final concentration, 2.5 µM) (manufactured by Stemgent) and XAV939 (final concentration, 5 µM) (manufactured by Merck). Subsequently, every other day, the medium was changed to RPMI 1640 medium (containing insulin). During the time period from Day 11 of induced differentiation (d11) to Day 15 (d15), beating cells started to appear.

### (Downstream Step of MC Protocol)

For the purpose of controlling the proportion of vascular mural cells (MCs) to be sufficient for forming an engineered cardiac tissue (ECT), only a part of the MC differentiation culture was used and MC differentiation was induced as needed. Specifically, the medium was changed to RPMI+FBS medium on Day 3 of induced differentiation (d3), and after this, the medium was changed every other day. The composition of the RPMI+FBS medium is as follows.
(RPMI+FBS medium)
RPMI 1640 medium (manufactured by Thermo Fisher)
L-glutamine (final concentration, 2 mM) (manufactured by Thermo Fisher)
Fetal bovine serum (FBS) (final concentration, 10 volume%)

### <<Flow Cytometry>>

In the following manner, flow cytometry was carried out. CV cells (CMs, ECs, MCs) derived from human iPS cells were incubated with Accumax for dissociation of the CV cells. Subsequently, surface-marker-specific antibodies listed below (only one of them or a combination of two of them, diluted to 1:100) were used for staining the CV cells.
(Surface-Marker-Specific Antibodies)
Phycoerythrin (PE)-conjugated anti-PDGFRβ antibody, clone 28d4 (manufactured by BD)
Allophycocyanin (APC)-conjugated anti-VE-cadherin antibody, clone 55-7hl (manufactured by BD)

Moreover, for the purpose of excluding dead cells from the analysis, the cells were stained with LIVE/DEAD Fixable Aqua Dead Cell Stain Kit (manufactured by Thermo Fisher). The cell surface markers were stained in PBS containing 5% FBS. Intracellular proteins were stained, in a state where the cells were fixed with PBS containing 4% paraformaldehyde (PFA). The cells were stained with APC-labeled anti-myocardial troponin T (cTnT) antibody (clone 13-11) (manufactured by Thermo Fisher), with the use of Zenon Technology (manufactured by Thermo Fisher) (1:50). Staining was performed in PBS containing 5% FBS and 0.75% saponin (manufactured by Sigma). The stained cells were analyzed with BD FACS Aria II (manufactured by BD) or CytoFLEX S (manufactured by Beckman Coulter). Data were collected from at least 10000 events. The data were analyzed with DIVA software (manufactured by BD) or CytExpert software (manufactured by Beckman Coulter).

### <<Preparation of Tissue Mold>>

A tissue mold was produced with polydimethylsiloxane (PDMS) (manufactured by Strex). After autoclaving, the tissue mold was coated with 1% Pluronic F127 for 1 hour. Prior to tissue molding, Pluronic F127 was removed and the tissue mold was rinsed sufficiently with PBS.

### <<Production of Engineered Cardiac Tissue (ECT) (Preparation Step)>>

For producing an ECT with or without ECs, cells differentiated in the CM+EC protocol (or cells differentiated in the CM protocol) and cells differentiated in the MC protocol were combined so that the final concentration (cell density) of MCs became 10 to 20%. The cells thus combined were mixed with acid-soluble rat tail collagen type I (manufactured by Sigma) and a matrix factor (Matrigel, manufactured by BD Biosciences).

Mixing the cells with the matrix was carried out in the following manner. (1) Firstly, 6,000,000 cells were suspended in a culture medium (high glucose modified Dulbecco essential medium, manufactured by Life Technologies) containing 20% fetal bovine serum (manufactured by Life Technologies). (2) Then, an acid-soluble type I collagen solution (2 mg/ml) (pH3) was neutralized on ice with an alkali buffer (0.2 M NaHCO₃, 0.2 M HEPES, 0.1 M NaOH). (3) Matrigel (15% of the total volume) was added to the neutralized collagen solution to obtain a matrix solution. (4) The above-mentioned cell suspension and the matrix solution were mixed together.

In the resulting mixture of the cells and the matrix (cell-matrix mixture), the final concentration of type I collagen was 0.67 mg/mL (total volume, 200 µL). The cell-matrix mixture was poured into the PDMS tissue mold coated with Pluronic F127, and the resultant was placed in a normal 12-well culture plate. It was followed by polymerization in a standard CO₂ incubator (37°C, 5% CO₂) for 60 minutes to form an ECT. After an ECT was thus formed, the tissue mold was immersed in a preculture medium. The composition of the preculture medium is described below. Prior to hydrostatic pressure stimulation, the formed ECT was incubated for 14 days (or 21 days). At this time, the medium was changed every other day.
(Composition of Preculture Medium)
Alpha minimum essential medium (αMEM, manufactured by Life Technologies)
10% FBS
5x10⁻⁵ M 2-mercaptoethanol (manufactured by Sigma)
100 U/mL penicillin-streptomycin (manufactured by Life Technologies)

By the above-described procedure, an ECT was prepared. The ECT corresponds to a sample containing cardiomyocytes.

### <<Hydrostatic Pressure Stimulation (Pressure-Applying Step)>>

Hydrostatic pressure stimulation was carried out in a MechanoCulture TR stimulation apparatus (MCTR, manufactured by CellScale) placed inside a tissue incubator. The tissue mold containing the ECT was transferred into an MCTR chamber, which was then filled with medium and set according to the hydrostatic pressure stimulation protocol described below (see Fig. 2). After hydrostatic pressure stimulation, the ECT was placed back into the 12-well culture plate.
(Hydrostatic Pressure Stimulation Protocol)
Applied pressure: 10 kPa, 50 kPa, or 100 kPa
Cycle of hydrostatic pressure stimulation: Compress duration for 0.2 s, hold duration for 0.1 s, recovery duration for 0.2 s, rest duration for 0.5 s
Mode of hydrostatic pressure stimulation: Continuous pressure application (24 hours per day) or intermittent pressure application (1 hour per day)
Number of days: 3 days or 8 days

### <<Histological Analysis and Fluorescence Analysis>>

The ECT was fixed with 4% PFA and embedded in paraffin. From the ECT thus fixed, a tissue fragment (thickness, 6 µm) was prepared. The tissue fragment was stained with hematoxylin-eosin, Sirius Red (SR), cTnT, and TUNEL.

For examination under a fluorescence microscope, the ECT was stained with DAPI (4',6-diamidino-2-phenylindole) (manufactured by Thermo Fisher) together with anti-cTnT antibody (manufactured by Abcam, ab45932) (diluted to 1:500) and anti-CD31 antibody (monoclonal mouse IgG1, clone 9G11) (manufactured by R&D) (diluted to 1:500). The single cells were stained with DAPI together with anti-cTnT antibody (mouse monoclonal antibody, MS-295-P1) (diluted to:1:500) and anti-TOMM20 antibody (manufactured by Abcam, ab78547) (1:500). As secondary antibodies, anti-mouse IgG antibody-Alexa 546 (manufactured by Thermo Fisher) and anti-rabbit IgG antibody-Alexa 488 (manufactured by Thermo Fisher) were used. An image of the ECT or the single cells was taken with an all-in-one fluorescence microscope system, Zeiss LSM880 (mean fluorescent intensity of TOMM20 with imageJ).

### <<Electric Stimulation Test>>

For electric stimulation testing of the ECT, a custom-made electric stimulation system (manufactured by Strex) equipped with an electric pulse generator was used. To the ECT incubated in a multi-well plate having 12 wells, two platinum electrodes were attached in parallel, and electric field electric stimulation was applied via the medium. Electric stimulation was applied with 4-ms pulse width, 1000-ms (1-Hz) interval, and 30-V voltage (between peaks), and the interval was gradually reduced to 500 ms (2 Hz). Capture of electric stimulation by the ECT was checked by simultaneous microscope examination.

### <<MUSCLEMOTION Analysis>>

MUSCLEMOTION is a versatile open software equipped with a video-based system to be used for assessing contractile function. The software was used according to the instructions from the manufacturer. More specifically, ImageJ software was used, and MUSCLEMOTION was installed as a plug-in. The amplitude of motion was used in analysis.

### <<Quantitative Analysis of Engineered Cardiac Tissue>>

ImageJ software was used for quantitative analysis of the ECT, such as the number of cells, cTnT-positive region, cell size, cell perimeter, TOMM20 mean fluorescent intensity (MFI), and time course of thickness of ECT (with ECs or without ECs).

### <<Calcium Transient>>

Calbryte (trade mark) 520AM (manufactured by AAT Bioquest, in a final concentration of 5 µM) loading solution containing 0.04% PF-127 was added to the tissue mold. The ECT, together with a dye loading buffer (a dye loading solution), was incubated in a 5% CO₂ incubator at 37°C for 60 minutes. The dye loading solution was replaced by FluoroBrite (trade mark) DMEM, prior to analysis. A fluorescence microscope (CKX53) equipped with a camera system (DP27) and a software (cellSens) was used for recording a video of the signal intensity through an FITC filter. Data analysis was carried out with the use of ImageJ software 52 equipped with "Plot Z-axis Profile" function with a manually selected ROI.

### <<Transmission Electron Microscopy Examination>>

The sample was fixed overnight with 2% glutaraldehyde+4% PFA/0.1-M phosphate buffer (pH7.2) at 4°C. The sample thus fixed was sent to the Electron Microscopy and Histology Facility, Kyoto University, for subsequent preparation, imaging, and data interpretation in a blind fashion.

### <<ECT Dissociation and Debris Removal Experiment>>

The ECT was rinsed twice with PBS, and then the ECT was incubated in a dissociation buffer. The composition of the dissociation buffer used here is as follows.

### (Composition of Dissociation Buffer)

120 mM NaCl
5.4 mM KCl
5 mM MgSO₄
5 mM Na-pyruvic acid
20 mM glucose
20 mM taurine
10 mM HEPES (pH6.9)
30 µM CaCl

Then, the cells derived from the dissociated ECT were subjected to debris removal experiment with the use of a debris removal solution (manufactured by Miltenyi Biotec), which was used according to the instructions from the manufacturer described below. (1) The cell suspension was centrifuged at 300×g for 10 minutes at 4°C. (2) The supernatant was completely removed by aspiration, and the cell suspension was carefully re-suspended in 500 µl of cold PBS. Subsequently, the same amount of cold debris removal solution was added thereto, gently overlaid on the cold PBS. (3) Centrifugation was carried out at 4°C and 3000×g for 10 minutes, with full acceleration and full brake. Three phases were formed, and the top two phases were completely removed by aspiration and discarded. Subsequently, the tube was filled with cold PBS and gently inverted three times. (4) Centrifugation was carried out at 4°C and 1000×g for 10 minutes with full acceleration and full brake, and the supernatant was completely removed by aspiration.

### <<Sepharose, Mito Stress Test>>

After the above-described ECT dissociation and debris removal, the purified single CMs were plated in a density of 100000 cells/well in a Seahorse XFp assay plate (manufactured by Agilent) coated with Matrigel, and allowed to proliferate for 3 days in an ordinary culture medium (αMEM). One hour prior to testing, the culture medium in the plate was changed to Seahorse assay medium (XF RPMI supplemented with 10 mM glucose, 1 mM pyruvic acid, and 2 mM glutamine), followed by incubation in the Seahorse assay medium in a CO₂-free incubator at 37°C. An XF cell mito stress test kit was used according to the instructions from the manufacturer, with the use of the below-listed compounds used in the following final concentrations.

### (Final Concentrations of Compounds Used Here)

Oligomycin, in 3 µM
2-[2-[4-(Trifluoromethoxy)phenyl]hydrazinylidene]-propanedinitrile (FCCP), in 2 µM
Rotenone/antimycin A, in 2 µM

### <<RNA Extraction, cDNA Synthesis, and Quantitative RT-PCR>>

Total RNA was isolated with Qiashredder (manufactured by Qiagen), and purified with RNeasy Mini Kit (manufactured by Qiagen). These processes were carried out according to the instructions from the manufacturer. In the purification treatment, DNase treatment was also carried out with RNase free DNase Set (manufactured by Qiagen). The yield and purity of RNA were measured with NanoDrop One (manufactured by Thermo Fisher Scientific). ReverTra Ace (registered trademark) qPCR RT Master Mix (FSQ-201, manufactured by TOYOBO) was used according to the written instructions from the manufacturer for synthesis of a first strand cDNA.

Out of each sample, 200 ng of the total RNA was used for reverse transcription into cDNA. The RNA was analyzed by quantitative real-time polymerase chain reaction (RT-PCR) on Applied Biosystems StepOne Plus (manufactured by Thermo Fisher Scientific) with the use of PowerUp (trade mark) SYBR Green Master Mix (manufactured by Thermo Fisher Scientific) according to the instructions from the manufacturer. Data analysis was carried out according to the fold change that was normalized to gene expression of glyceraldehyde-3-phosphate dehydrogenase (GAPDH).

### <<MtDNA PCR>>

From the ECT, mitochondrial DNA (MtDNA) was extracted with the use of a DNA digestion buffer (a buffer prepared by adding proteinase K to Qiagen Cell Lysis solution in 1:100). The MtDNA was analyzed by quantitative RT-PCR on Applied Biosystems StepOne Plus (manufactured by Thermo Fisher Scientific) with the use of PowerUp (trade mark) SYBR Green Master Mix (manufactured by Thermo Fisher Scientific) according to the instructions from the manufacturer. The primer for MtDNA is mitochondrial gene NADH dehydrogenase 1 (ND1). The amount of expression of MtDNA was normalized to the expression of RNA18S5 gene.

### <<Single-Cell RNA Sequencing>>

After the above-described ECT dissociation and debris removal, the purified single CMs were subjected to single-cell RNA sequencing (scRNA-seq) on Chromium Single-Cell Platform (manufactured by 10×Genomics). Cell barcoding, cDNA synthesis, and library construction were carried out with the use of Single Cell Gene Expression 3' Kit version 2 (manufactured by 10×Genomics, PN120237) or Single Cell Gene Expression 3' Kit version 3 (manufactured by 10×Genomics, PN1000075), which were used according to the instructions from the manufacturer. Data of scRNA-seq were analyzed with the use of loupe browser 6, and pathway analysis was carried out with the use of iDEP0.96 (ShinyGO 0.76.3).

### <<Animal Model Preparation and Transplantation>>

For transplantation, 10- to 13-week-old male athymic nude rats (F344/N Jcl-rnu/rnu, manufactured by CLEA Japan, Inc.) were used. By the following procedure, myocardial infarction (MI) model rats were prepared. Firstly, the left chest of each rat was opened and the heart was exposed. The left anterior descending branch (a coronary artery) of the exposed heart was ligated, and thereby an MI model rat was prepared.

Rats the heart of which was found to have a left ventricular fractional shortening (FS) of less than 40% in echocardiography (an echocardiogram) performed immediately before MI induction were used in the experiment described below. One week after MI induction, a cell sheet was transplanted. The rats were divided randomly into three groups (rats with MCTR training and ECT transplantation (MCTR group), rats without MCTR training and with ECT transplantation (Control group), and rats with sham surgery (Placebo group or Sham group)). Into the heart of the rat, an ECT in the form of a stack of three layers was transplanted. Echocardiography was carried out before MI model preparation (PreMI), 1 week after MI model preparation (immediately before transplantation, PreTx), 2 weeks after transplantation (Tx_2w), and 4 weeks after transplantation (Tx_4w).

### <<Statistical Analysis>>

Data were in the form of average values±SD. Differences between the experiment groups were evaluated by one way repeated measures ANOVA with GraphPad Prism (9.0) as well as t-test (nonparametric test). P value<0.05 was regarded as significant in all statistic tests.

### <<Experiment Results>>

### <<Preparation of Engineered Cardiac Tissue Derived from Human iPS Cells>>

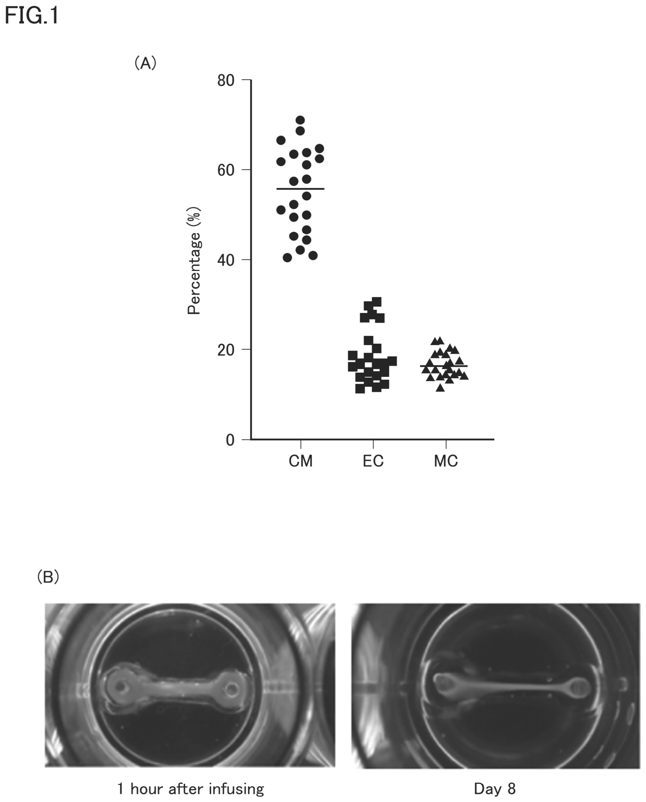

According to the above-described experiment procedure, cardiovascular cells obtained by induced differentiation from human iPS cells (201B6) were co-cultured with extracellular matrix protein on the tissue mold, to prepare an engineered cardiac tissue (ECT) from human iPS cells (B of Fig. 1). The cell composition of the ECT immediately after preparation is shown in A of Fig. 1. In A of Fig. 1, the horizontal axis represents the type of cells constituting the ECT, and the vertical axis represents the ratio of each cell type in the ECT. The results shown in A of Fig. 1 indicate that about 60% of the ECT was made up of CMs, about 20% was ECs, and about 20% was MCs. It should be noted that the ECT used in later experiments is an ECT derived from human iPS cell line (201B6).

### <<Hydrostatic Pressure Stimulation of ECT>>

Firstly, optimization of the pattern of pressure application to the ECT was attempted. The heart is constantly exposed to continuous afterload pressure. In this regard, there is a report suggesting that a rapid increase of afterload pressure in a ventricle due to ascending aortic constriction (AAC) as well as a relaxation of the increased afterload due to removal of aortic constriction and a return to the normal state can further stimulate re-entry of cardiomyocytes to the cell cycle (or increase the number of cardiomyocytes in the heart).

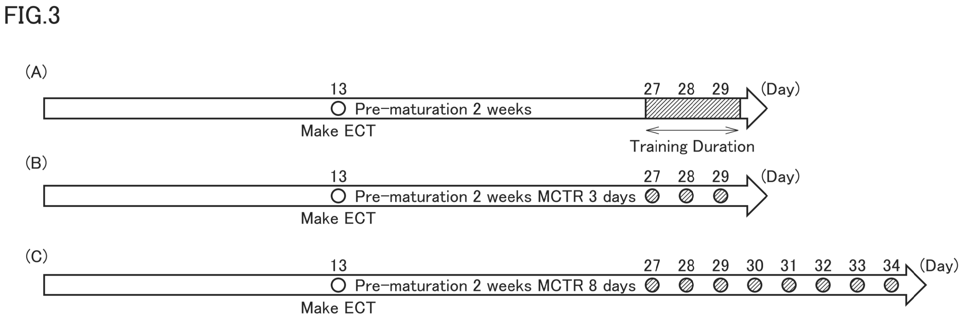

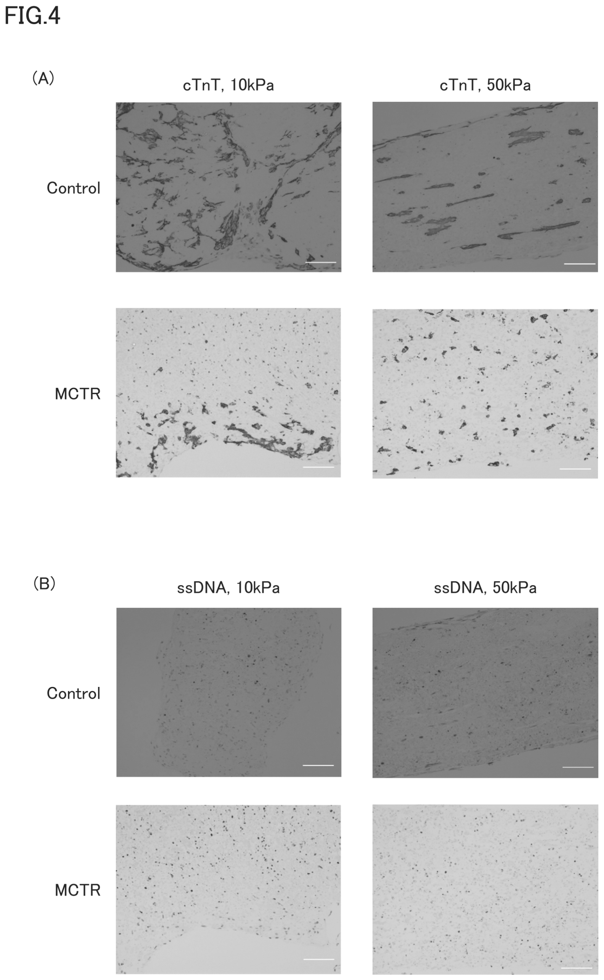

For clarifying the influence of continuous pressure overload, continuous pressure stimulation was applied under a physiological pressure condition (10 KPa; corresponding to about 75 mmHg) and a non-physiological high pressure condition (50 KPa; corresponding to about 375 mmHg) (upper diagram in Fig. 3). Histological evaluation found almost no cTnT staining, indicating that continuous pressure application can damage tissue without an increase of cardiomyocytes in the ECT (Fig. 4).

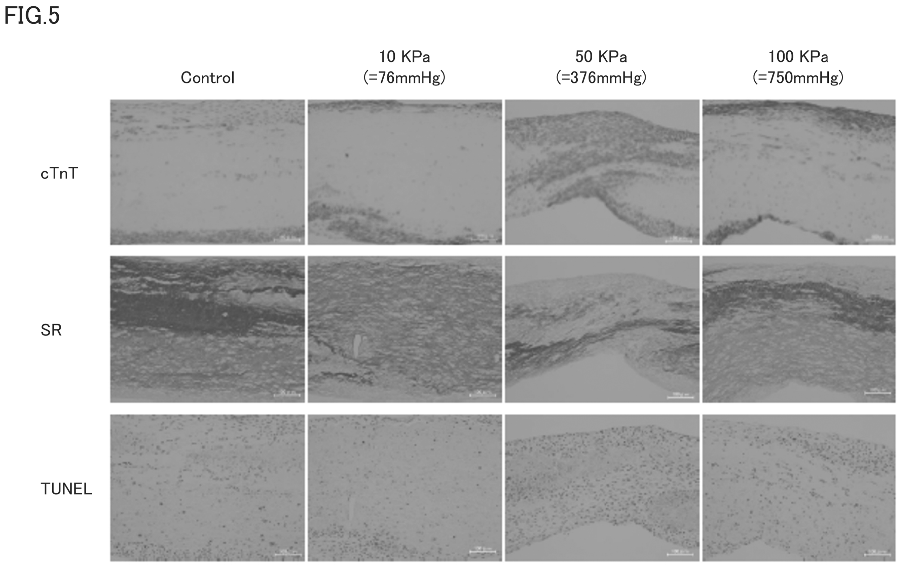

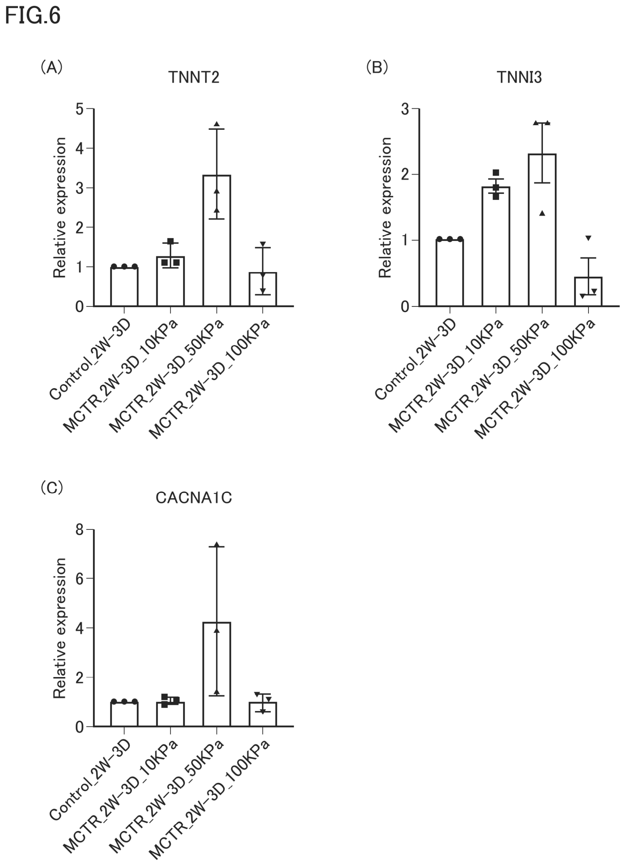

In consideration of the results in Fig. 4, intermittent pressure application at 10 KPa, 50 KPa, and 100 KPa (training for 3 days, 1 hour per day) was carried out (central diagram in Fig. 3). It was demonstrated that when applied at 50 KPa, the ECT exhibited relatively high cTnT-positive cardiomyocyte components, relatively high living cell density, as well as relatively high expression of TNNT2, TNNI3, and CACNA1C genes associated with relatively high collagen alignment (Fig. 5, Fig. 6). This indicates that the hydrostatic pressure stimulation caused maturation of cardiomyocytes in the ECT. In contrast, no positive influence was observed in the ECT applied pressure at 10 KPa or 100 KPa (Fig. 5, Fig. 6). Here, the expression "MCTR_2W _3D _10 KPa" in Fig. 6 means a sample that was intermittently applied a pressure of 10 kPa for 3 days after two-week incubation of the formed ECT. Moreover, the expression "Control_2W_3D" means a sample after two-week incubation of the formed ECT. The same style applies to the other expressions.

For evaluating contractile function (beating function) of the ECT, MUSCLEMOTION was used. MUSCLEMOTION is a multirole video-based system for quantification of the amplitude of motion of ECT. As for the control group that did not receive hydrostatic pressure stimulation, the amplitude of motion in the entire beating cycle decreased as the frequency of external electric stimulation increased, both before and after the period corresponding to training (the test period), indicating a strong negative correlation. On the other hand, as for the MCTR group that received hydrostatic pressure stimulation, it was negative before the training, and then after the training, as the electric stimulation increased from 1000 ms (1 Hz) to 800 ms (1.25 Hz), the amplitude of motion in the entire beating cycle clearly increased. These results demonstrate that intermittent stimulation of the ECT with a non-physiological level of hydrostatic pressure at 50 KPa induces a positive relationship between contraction intensity and contraction frequency (positive force frequency relationship, FFR).

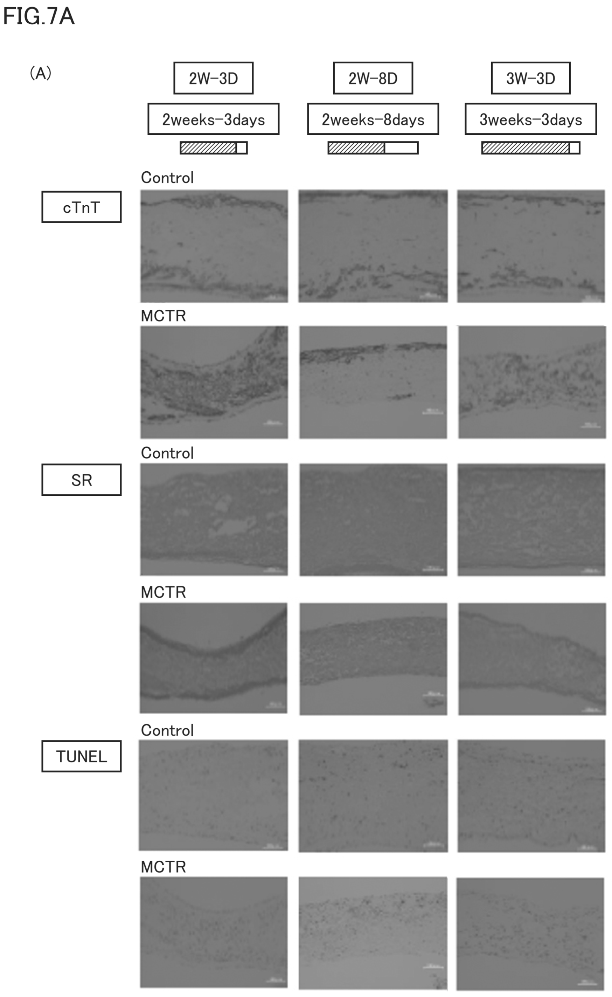

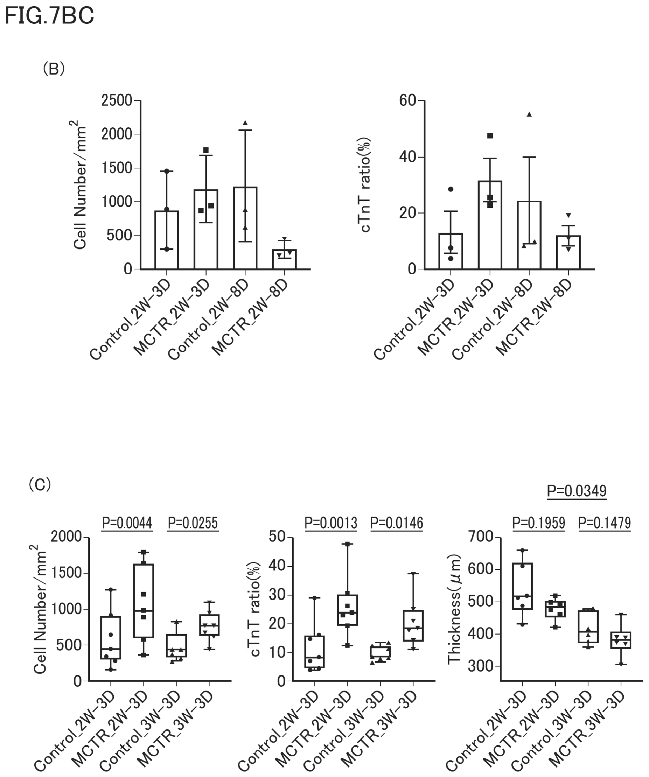

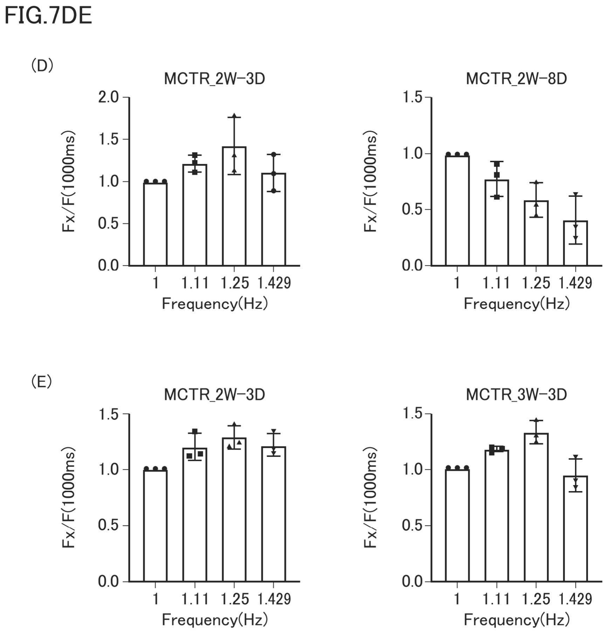

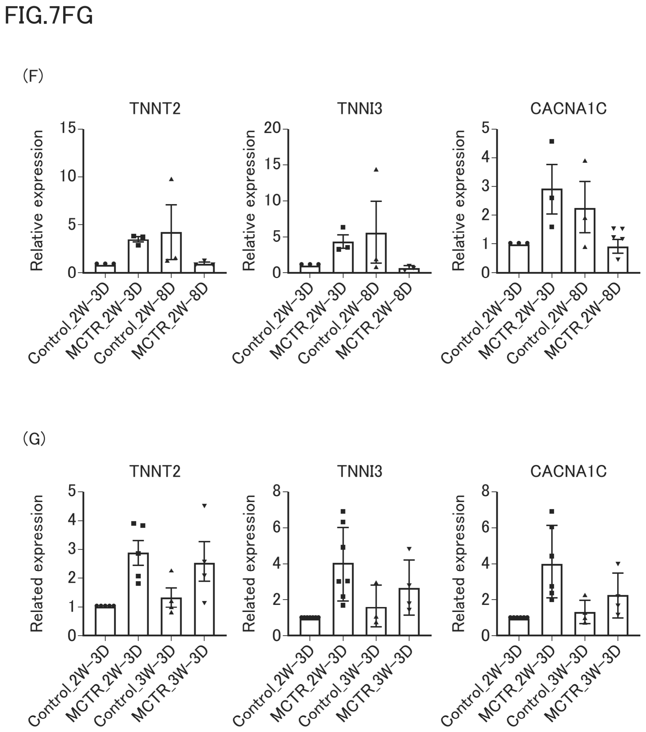

For further optimization of the method of hydrostatic pressure stimulation, the number of days to apply intermittent stimulation was investigated. A 3-day incubation (1 hour/day) group and an 8-day incubation (1 hour/day) group were compared, and corresponding control groups (a control 3-day group and a control 8-day group) were also established (central diagram and lower diagram in Fig. 3). These four groups were subjected to evaluation of structural function by tissue staining (Fig. 7A, B of Fig. 7BC), evaluation of contractile force (D of Fig. 7DE), and evaluation of gene expression by RT-qPCR (F of Fig. 7FG). The results indicated that as compared to 8-day intermittent hydrostatic pressure stimulation, 3-day intermittent hydrostatic pressure stimulation was effective for ECT maturation.

In the above-described procedures, hydrostatic pressure stimulation was applied after 2-week incubation of the formed ECT. It is known that early-stage cardiomyocytes shortly after induced differentiation from human iPS cells are sensitive to electric stimulation training as compared to cardiomyocytes in later phases of induced differentiation. This suggests that the timing to start the physical training is important for tissue maturation. In light of this, a group incubated for 2 weeks before hydrostatic pressure stimulation and a group incubated for 3 weeks before hydrostatic pressure stimulation were compared by histological staining, MUSCLEMOTION analysis, and RT-qPCR (Fig. 7A, C of Fig. 7BC, E of Fig. 7DE, G of Fig. 7FG). The results of histological staining demonstrated that both groups had a higher cTnT-positive ratio and a higher cell viability than the control, and in particular, the group incubated for 2 weeks before hydrostatic pressure stimulation had significantly higher results (Fig. 7A, C of Fig. 7BC). An FFR was achieved in both the group incubated for 2 weeks before hydrostatic pressure stimulation and the group incubated for 3 weeks before hydrostatic pressure stimulation (E of Fig. 7DE). The expression levels of TNNT, TNNI3, TNNI3/TNNI1, and CACNA1C increased in both groups. From these results, it was indicated that hydrostatic pressure stimulation of the ECT had positive effects to both the group incubated for 2 weeks before hydrostatic pressure stimulation and the group incubated for 3 weeks before hydrostatic pressure stimulation, and in particular, the group incubated for 2 weeks before hydrostatic pressure stimulation exhibited significant effects (G of Fig. 7FG).

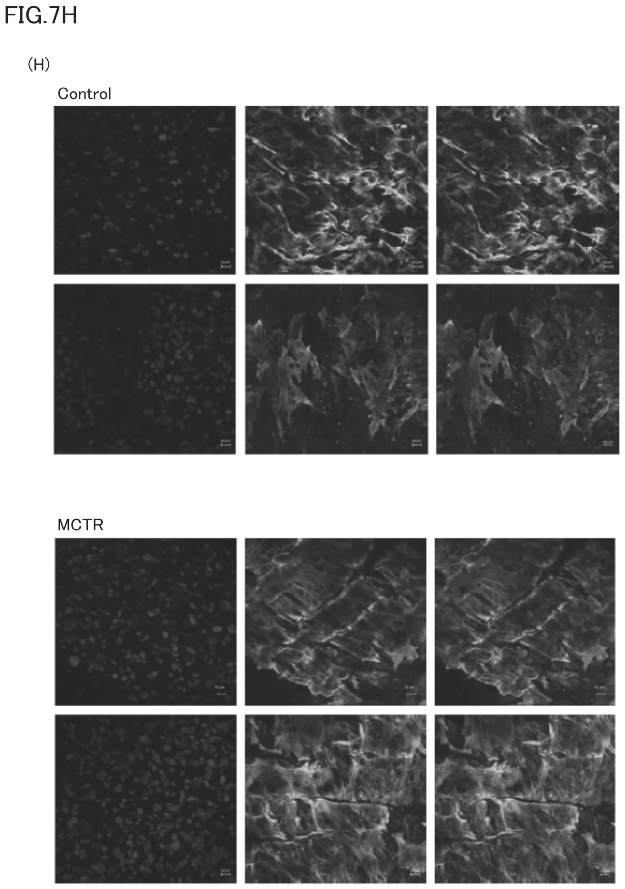

In terms of how the cardiomyocytes were arranged in the ECT, the control group and the MCTR group were compared. Results are shown in Fig. 7H. Fig. 7H shows photographs of results of fluorescence analysis of the ECT. From the results shown in Fig. 7H, it was indicated that in the ECT of the MCTR group, as compared to the control group (Control), cardiomyocytes are aligned in the same direction. It is known that cardiomyocytes are aligned in the same direction in the cardiac tissue of a matured living body and thereby the cardiomyocytes can contract in the same direction to exhibit contractile force. Due to the MCTR training, the cardiomyocytes became aligned in a similar manner to those in cardiac muscle in matured cardiac tissue (namely, they became aligned in the same direction), indicating that they can exhibit similar contractile function to those in cardiac tissue of a living body.

The results indicated that cardiomyocytes in the ECT became matured due to hydrostatic pressure stimulation. It was also indicated that it is optimum to apply the hydrostatic pressure stimulation under conditions at 50 kPa and 1 hour/day for 3 days, after 2-week incubation performed prior to the hydrostatic pressure stimulation.

### <<Evaluation of FFR in ECT>>

The ECT that received hydrostatic pressure stimulation training under the above-described optimum conditions was evaluated in terms of FFR. As a result, the ECT exhibited a positive FFR, indicating that the tissue was matured (Fig. 8AB). The proportion of cardiomyocytes was higher in the group with a positive FFR than in the group with a negative FFR. The expression levels of TNNT, TNNI3, TNNI3/TNNI1, and CACNA1C were higher in the group with a positive FFR. However, no significant difference from the group with a negative FFR was observed. From the PCR experiment data (Fig. 7FG), it was predicted that one of the mechanisms of tissue maturation can be an improved calcium handling. Calcium transient can be visualized with calbryte 520 (manufactured by AAT bioquest (ABD)). Representative spontaneous calcium transients in the control group and the MCTR group are shown in Fig. 8CDEF. Many different parameters were measured, and it was indicated that calcium transient markedly increased in the MCTR group. The peak of calcium flux increased beyond 100%. The amount of expression of PLN and SERCA2 markedly increased in the MCTR group (F of Fig. 8CDEF), indicating a high calcium handling capacity in the ECT that received MCTR training. This indicates that cardiomyocytes in the ECT became matured due to hydrostatic pressure stimulation.

It was also predicted that another mechanism of tissue maturation caused by MCTR training can be mitochondrial function. Mitochondrial function was evaluated with Mito Stress Test manufactured by Seahorse, and as a result, the maximum oxygen consumption rate (OCR) in the ECT that received MCTR training was much higher than in the control group (Fig. 9AB). The ECT was examined under a TEM, and as a result, more mitochondria were observed in the MCTR group than in the control group (C of Fig. 9CDE). It was found that these mitochondria were located between sarcomeres (C of Fig. 9CDE). Furthermore, anti-TOMM20 antibody was checked by confocal imaging, and it was found that the area positive for TOMM20 (a mitochondrial marker) was larger in cardiomyocytes in the ECT that received MCTR training (D of Fig. 9CDE, F of Fig. FG). Mitochondrial DNA was quantitatively assessed by qPCR (E of Fig. 9CDE). The results indicated that the MCTR training increased the amount of mitochondria in cardiomyocytes and enabled production of more energy (ATP). G of Fig. 9FG shows graphs of cell size (left), perimeter (central), and perimeter/area ratio (right). As cardiomyocytes mature, the size becomes greater and thereby the perimeter becomes longer. Moreover, as cardiomyocytes mature, the structure becomes more complex and thereby the cell shape becomes more complex. The phenomenon of the cell shape becoming more complex is backed by the increase of the perimeter/area ratio. That is, from the results of G of Fig. 9FG, it was indicated that the MCTR training matured individual cardiomyocytes also in terms of shape.

For comprehensive understanding of the mechanism of tissue maturation due to MCTR training, single cell RNA-seq was carried out (Fig. 10AB, Fig. 10CD). As a result, it was indicated from different aspects that maturation of the function of cardiomyocytes in the engineered cardiac tissue proceeded due to the MCTR training, and it was demonstrated that a mechanism of this phenomenon is involved with facilitated oxidative phosphorylation which is advantageous to energy production.

Formation of vascular networks is a basic factor for establishment of matured cardiac tissue. It is considered that endothelial cells are sensitive to hydrostatic pressure. In light of this, responses of ECs to MCTR were further investigated. An ECT with ECs and an ECT without ECs were prepared (A of Fig. 11ABC). These ECTs were different from each other at the time of production. They were also different in terms of the extent of contraction one hour after the ECT was compressed, as well as in terms of subsequent beating (B and C of Fig. 11ABC). The without-EC ECT group exhibited a negative FFR (D of Fig. 11DEF). In the without-EC ECT group, the expression levels of TNNT, TNNI3, TNNI3/TNNI1, CACNA1C, PLN, SERCA2, MHY7, and MHY6 decreased due to the MCTR training (E of Fig. 11DEF). Confocal imaging of the ECT after tissue removal showed vascular networks formed in the with-EC ECT group (Fig. 12). In contrast, in the without-EC ECT group, formation of vascular networks was not observed (F of Fig. 11DEF). The results demonstrated that formation of vascular networks was facilitated when endothelial cells were present in the ECT.

In the experiment corresponding to Fig. 12, when post-training rocking incubation was changed to 1 week, a vascular network formed of CD31-positive vascular endothelial cells was observed in the ECT "with vascular endothelial cells" (with ECs) and with MCTR training (Fig. 13). To this ECT in which a vascular network was observed, electric stimulation was applied with the stimulation frequency gradually increased starting from 1000 ms, and as a result, a positive FFR was exhibited (Fig. 14, lower diagram) (this result suggests that the subject is a mature tissue). In contrast, in the control group "with vascular endothelial cells" (with ECs) and without MCTR training, a negative force-frequency relationship (FFR) was exhibited (Fig. 14, upper diagram) (this result suggests that the subject is an immature tissue).

### <<Transplantation Experiment in MI Model Rat>>

With the use of MI model rats, ECT transplantation experiment was carried out. Used here were rats the heart of which was found to have a left ventricular fractional shortening (FS) of less than 40% in echocardiography (an echocardiogram) performed immediately before MI induction. One week after MI induction, a cell sheet (an ECT in the form of a stack of three layers) was transplanted. The rats were divided randomly into three groups as described below.

### (Three Groups)

MCTR group: Rats with MCTR training and with ECT transplantation
Control group: Rats without MCTR training and with ECT transplantation
Sham group: Rats with sham surgery

Echocardiography was carried out before MI model preparation (PreMI), 1 week after MI model preparation (immediately before transplantation, PreTx), 2 weeks after transplantation (Tx_2w), and 4 weeks after transplantation (Tx_4w). Results are shown in Fig. 15. The results shown in Fig. 15 indicate that cardiac function in the MCTR group was high (in particular, 2 weeks after transplantation (Tx_2w)).

Although the embodiment and examples of the present invention are described above, it is originally planned that certain configurations of the embodiment and examples can be combined as appropriate.

The embodiment and examples disclosed herein are illustrative and non-restrictive in any respect. The scope of the present invention is defined by the terms of the claims, not by the embodiment and examples, and intended to encompass all modifications and variations equivalent in meaning and scope to the claims.

## Claims

1. A method of producing matured cardiomyocytes, the method comprising:
a preparation step to prepare a sample containing immature cardiomyocytes; and
a pressure-applying step to apply hydrostatic pressure continuously or intermittently to the sample.

2. The method of producing matured cardiomyocytes according to claim 1, wherein the hydrostatic pressure applied in the pressure-applying step is from 10 kPa to 100 kPa.

3. The method of producing matured cardiomyocytes according to claim 1 or 2, wherein in the pressure-applying step, the hydrostatic pressure is applied intermittently at a frequency from 1 hour to 12 hours per day.

4. The method of producing matured cardiomyocytes according to any one of claims 1 to 3, wherein the pressure-applying step is implemented for a period from 3 days to 8 days.

5. The method of producing matured cardiomyocytes according to any one of claims 1 to 4, wherein the sample further contains vascular mural cells.

6. The method of producing matured cardiomyocytes according to any one of claims 1 to 5, wherein the sample further contains vascular endothelial cells.

7. The method of producing matured cardiomyocytes according to any one of claims 1 to 6, wherein the sample is an engineered cardiac tissue derived from iPS cells.

8. An engineered cardiac tissue containing cardiomyocytes, wherein the cardiomyocytes include TNNI3-positive cells.
